# EUROPEAN PATENT APPLICATION

(11) **EP 3 984 554 A1**
(43) Date of publication of application: **20.04.2022**
(21) Application number: 20822287.7
(22) Date of filing: 10.06.2020
(51) Int. Cl.: A61K 39/395, A61P 37/06, A61P 37/08, C07K 16/28, C07K 16/46

(54) **IMMUNOSUPPRESSION AGENT**

(30) Priority: 11.06.2019 JP 2019108906
(71) Applicant: ONO Pharmaceutical Co., Ltd., Osaka-shi, Osaka 541-8526 (JP); Tokushima University, Tokushima-shi, Tokushima 770-8501 (JP)
(72) Inventor: OKAZAKI, Taku, Tokushima-shi, Tokushima 770-8501 (JP); SUGIURA, Daisuke, Tokushima-shi, Tokushima 770-8501 (JP); OKAZAKI, Il-mi, Tokushima-shi, Tokushima 770-8501 (JP); SHIBAYAMA, Shiro, Tsukuba-shi, Ibaraki 300-4247 (JP)
(74) Representative: J A Kemp LLP
(86) International application number: PCT/JP2020/022882
(87) International publication number: WO 2020/250940

(57) **Abstract**

The present disclosure provides an immunosuppressant containing a bispecific molecule including a first binding site that specifically binds to LAG3 and a second binding site that specifically binds to CD3 or CD8.

SEQUENCE LISTING

<110>  ONO PHARMACEUTICAL CO., LTD.
       TOKUSHIMA UNIVERSITY

<120>  IMMUNOSUPPRESSIVE AGENT

<130>  675574

<150>  JP 2019-108906
<151>  2019-06-11

<160>  34

<170>  PatentIn version 3.5

<210>  1
<211>  525
<212>  PRT
<213>  Homo sapiens

<400>  1

Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln
        115                 120                 125

Arg Gly Asp Phe Ser Leu Trp Leu Arg Pro Ala Arg Arg Ala Asp Ala
    130                 135                 140

Gly Glu Tyr Arg Ala Ala Val His Leu Arg Asp Arg Ala Leu Ser Cys
145                 150                 155                 160

Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
            180                 185                 190

Arg Pro Asp Arg Pro Ala Ser Val His Trp Phe Arg Asn Arg Gly Gln
        195                 200                 205

Gly Arg Val Pro Val Arg Glu Ser Pro His His His Leu Ala Glu Ser
    210                 215                 220

Phe Leu Phe Leu Pro Gln Val Ser Pro Met Asp Ser Gly Pro Trp Gly
225                 230                 235                 240

Cys Ile Leu Thr Tyr Arg Asp Gly Phe Asn Val Ser Ile Met Tyr Asn
                245                 250                 255

Leu Thr Val Leu Gly Leu Glu Pro Pro Thr Pro Leu Thr Val Tyr Ala
            260                 265                 270

Gly Ala Gly Ser Arg Val Gly Leu Pro Cys Arg Leu Pro Ala Gly Val
        275                 280                 285

Gly Thr Arg Ser Phe Leu Thr Ala Lys Trp Thr Pro Pro Gly Gly Gly
    290                 295                 300

Pro Asp Leu Leu Val Thr Gly Asp Asn Gly Asp Phe Thr Leu Arg Leu
305                 310                 315                 320

Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
            340                 345                 350

Val Thr Pro Lys Ser Phe Gly Ser Pro Gly Ser Leu Gly Lys Leu Leu
        355                 360                 365

Cys Glu Val Thr Pro Val Ser Gly Gln Glu Arg Phe Val Trp Ser Ser
    370                 375                 380

Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Ile Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525

<210> 2
<211> 521
<212> PRT
<213> Mus musculus

<400> 2

Met Arg Glu Asp Leu Leu Leu Gly Phe Leu Leu Leu Gly Leu Leu Trp
1               5                   10                  15

Glu Ala Pro Val Val Ser Ser Gly Pro Gly Lys Glu Leu Pro Val Val
            20                  25                  30

Trp Ala Gln Glu Gly Ala Pro Val His Leu Pro Cys Ser Leu Lys Ser
        35                  40                  45

Pro Asn Leu Asp Pro Asn Phe Leu Arg Arg Gly Gly Val Ile Trp Gln
    50                  55                  60

His Gln Pro Asp Ser Gly Gln Pro Thr Pro Ile Pro Ala Leu Asp Leu
65                  70                  75                  80

His Gln Gly Met Pro Ser Pro Arg Gln Pro Ala Pro Gly Arg Tyr Thr
                85                  90                  95

Val Leu Ser Val Ala Pro Gly Gly Leu Arg Ser Gly Arg Gln Pro Leu
            100                 105                 110

His Pro His Val Gln Leu Glu Glu Arg Gly Leu Gln Arg Gly Asp Phe
        115                 120                 125

Ser Leu Trp Leu Arg Pro Ala Leu Arg Thr Asp Ala Gly Glu Tyr His
    130                 135                 140

Ala Thr Val Arg Leu Pro Asn Arg Ala Leu Ser Cys Ser Leu Arg Leu
145                 150                 155                 160

Arg Val Gly Gln Ala Ser Met Ile Ala Ser Pro Ser Gly Val Leu Lys
                165                 170                 175

Leu Ser Asp Trp Val Leu Leu Asn Cys Ser Phe Ser Arg Pro Asp Arg
            180                 185                 190

Pro Val Ser Val His Trp Phe Gln Gly Gln Asn Arg Val Pro Val Tyr
        195                 200                 205

Asn Ser Pro Arg His Phe Leu Ala Glu Thr Phe Leu Leu Leu Pro Gln
    210                 215                 220

Val Ser Pro Leu Asp Ser Gly Thr Trp Gly Cys Val Leu Thr Tyr Arg
225                 230                 235                 240

Asp Gly Phe Asn Val Ser Ile Thr Tyr Asn Leu Lys Val Leu Gly Leu
                245                 250                 255

Glu Pro Val Ala Pro Leu Thr Val Tyr Ala Ala Glu Gly Ser Arg Val
            260                 265                 270

Glu Leu Pro Cys His Leu Pro Pro Gly Val Gly Thr Pro Ser Leu Leu
        275                 280                 285

Ile Ala Lys Trp Thr Pro Pro Gly Gly Gly Pro Glu Leu Pro Val Ala
    290                 295                 300

Gly Lys Ser Gly Asn Phe Thr Leu His Leu Glu Ala Val Gly Leu Ala
305                 310                 315                 320

Gln Ala Gly Thr Tyr Thr Cys Ser Ile His Leu Gln Gly Gln Gln Leu
                325                 330                 335

Asn Ala Thr Val Thr Leu Ala Val Ile Thr Val Thr Pro Lys Ser Phe
            340                 345                 350

Gly Leu Pro Gly Ser Arg Gly Lys Leu Leu Cys Glu Val Thr Pro Ala
        355                 360                 365

Ser Gly Lys Glu Arg Phe Val Trp Arg Pro Leu Asn Asn Leu Ser Arg
    370                 375                 380

Ser Cys Pro Gly Pro Val Leu Glu Ile Gln Glu Ala Arg Leu Leu Ala
385                 390                 395                 400

Glu Arg Trp Gln Cys Gln Leu Tyr Glu Gly Gln Arg Leu Leu Gly Ala
                405                 410                 415

Thr Val Tyr Ala Ala Glu Ser Ser Ser Gly Ala His Ser Ala Arg Arg
            420                 425                 430

Ile Ser Gly Asp Leu Lys Gly Gly His Leu Val Leu Val Leu Ile Leu
        435                 440                 445

Gly Ala Leu Ser Leu Phe Leu Leu Val Ala Gly Ala Phe Gly Phe His
    450                 455                 460

Trp Trp Arg Lys Gln Leu Leu Leu Arg Arg Phe Ser Ala Leu Glu His
465                 470                 475                 480

Gly Ile Gln Pro Phe Pro Ala Gln Arg Lys Ile Glu Glu Leu Glu Arg
                485                 490                 495

Glu Leu Glu Thr Glu Met Gly Gln Glu Pro Glu Pro Glu Pro Glu Pro
            500                 505                 510

Gln Leu Glu Pro Glu Pro Arg Gln Leu
        515                 520

<210>  3
<211>  207
<212>  PRT
<213>  Homo sapiens

<400>  3

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  4
<211>  189
<212>  PRT
<213>  Mus musculus

<400>  4

Met Arg Trp Asn Thr Phe Trp Gly Ile Leu Cys Leu Ser Leu Leu Ala
1               5                   10                  15

Val Gly Thr Cys Gln Asp Asp Ala Glu Asn Ile Glu Tyr Lys Val Ser
            20                  25                  30

Ile Ser Gly Thr Ser Val Glu Leu Thr Cys Pro Leu Asp Ser Asp Glu
        35                  40                  45

Asn Leu Lys Trp Glu Lys Asn Gly Gln Glu Leu Pro Gln Lys His Asp
    50                  55                  60

Lys His Leu Val Leu Gln Asp Phe Ser Glu Val Glu Asp Ser Gly Tyr
65                  70                  75                  80

Tyr Val Cys Tyr Thr Pro Ala Ser Asn Lys Asn Thr Tyr Leu Tyr Leu
                85                  90                  95

Lys Ala Arg Val Cys Glu Tyr Cys Val Glu Val Asp Leu Thr Ala Val
            100                 105                 110

Ala Ile Ile Ile Ile Val Asp Ile Cys Ile Thr Leu Gly Leu Leu Met
        115                 120                 125

Val Ile Tyr Tyr Trp Ser Lys Asn Arg Lys Ala Lys Ala Lys Pro Val
    130                 135                 140

Thr Arg Gly Thr Gly Ala Gly Ser Arg Pro Arg Gly Gln Asn Lys Glu
145                 150                 155                 160

Arg Pro Pro Pro Val Pro Asn Pro Asp Tyr Glu Pro Ile Arg Lys Gly
                165                 170                 175

Gln Arg Asp Leu Tyr Ser Gly Leu Asn Gln Arg Ala Val
            180                 185

<210>  5
<211>  235
<212>  PRT
<213>  Homo sapiens

<400>  5

<210>  6
<211>  247
<212>  PRT
<213>  Mus musculus

<400>  6

Met Ala Ser Pro Leu Thr Arg Phe Leu Ser Leu Asn Leu Leu Leu Leu
1               5                   10                  15

Gly Glu Ser Ile Ile Leu Gly Ser Gly Glu Ala Lys Pro Gln Ala Pro
            20                  25                  30

Glu Leu Arg Ile Phe Pro Lys Lys Met Asp Ala Glu Leu Gly Gln Lys
        35                  40                  45

Val Asp Leu Val Cys Glu Val Leu Gly Ser Val Ser Gln Gly Cys Ser
    50                  55                  60

Trp Leu Phe Gln Asn Ser Ser Ser Lys Leu Pro Gln Pro Thr Phe Val
65                  70                  75                  80

Val Tyr Met Ala Ser Ser His Asn Lys Ile Thr Trp Asp Glu Lys Leu
                85                  90                  95

Asn Ser Ser Lys Leu Phe Ser Ala Met Arg Asp Thr Asn Asn Lys Tyr
            100                 105                 110

Val Leu Thr Leu Asn Lys Phe Ser Lys Glu Asn Glu Gly Tyr Tyr Phe
        115                 120                 125

Cys Ser Val Ile Ser Asn Ser Val Met Tyr Phe Ser Ser Val Val Pro
    130                 135                 140

Val Leu Gln Lys Val Asn Ser Thr Thr Thr Lys Pro Val Leu Arg Thr
145                 150                 155                 160

Pro Ser Pro Val His Pro Thr Gly Thr Ser Gln Pro Gln Arg Pro Glu
                165                 170                 175

Asp Cys Arg Pro Arg Gly Ser Val Lys Gly Thr Gly Leu Asp Phe Ala
            180                 185                 190

Cys Asp Ile Tyr Ile Trp Ala Pro Leu Ala Gly Ile Cys Val Ala Leu
        195                 200                 205

Leu Leu Ser Leu Ile Ile Thr Leu Ile Cys Tyr His Arg Ser Arg Lys
    210                 215                 220

Arg Val Cys Lys Cys Pro Arg Pro Leu Val Arg Gln Glu Gly Lys Pro
225                 230                 235                 240

Arg Pro Ser Glu Lys Ile Val
                245

<210> 7
<211> 118
<212> PRT
<213> Mus musculus

<400> 7

Val Gln Leu Gln Gln Ser Gly Ala Glu Leu Val Arg Pro Gly Ala Leu
1               5                   10                  15

Val Lys Leu Ser Cys Lys Ala Ser Gly Phe Asn Ile Lys Asp Tyr Tyr
            20                  25                  30

Met His Trp Val Lys Gln Arg Pro Glu Gln Gly Leu Glu Trp Ile Gly
        35                  40                  45

Trp Ile Asp Pro Glu Asn Gly Asn Thr Ile Tyr Asp Pro Lys Phe Gln
    50                  55                  60

Asp Lys Ala Ser Leu Thr Ala Asp Thr Ser Ser Asn Thr Ala Tyr Leu
65                  70                  75                  80

Gln Leu Ser Ser Leu Thr Ser Glu Asp Thr Ala Val Tyr Tyr Cys Ala
                85                  90                  95

Pro Glu Arg Gly Tyr Asp Tyr Ala Met Asp Tyr Trp Gly Gln Gly Thr
            100                 105                 110

Ser Val Thr Val Ser Ser
        115

<210>  8
<211>  107
<212>  PRT
<213>  Mus musculus

<400>  8

Ile Val Leu Thr Gln Ser Pro Ala Thr Leu Ser Val Thr Pro Gly Asp
1               5                   10                  15

Ser Val Ser Leu Ser Cys Arg Ala Ser Gln Ser Ile Ser Asn Asn Leu
            20                  25                  30

His Trp Tyr Gln Gln Lys Ser His Glu Ser Pro Arg Leu Leu Ile Lys
        35                  40                  45

Tyr Ala Ser Gln Ser Ile Ser Gly Ile Pro Ser Arg Phe Ser Gly Ser
    50                  55                  60

Gly Ser Gly Thr Asp Phe Thr Leu Ser Ile Asn Ser Val Glu Thr Glu
65                  70                  75                  80

Asp Phe Gly Met Tyr Phe Cys Gln Gln Ser Asn Ser Trp Pro Gln Tyr
                85                  90                  95

Thr Phe Gly Gly Gly Thr Lys Leu Glu Ile Lys
            100                 105

<210>  9
<211>  5
<212>  PRT
<213>  Mus musculus

<400>  9

Asp Tyr Tyr Met His
1               5

<210>  10
<211>  17
<212>  PRT
<213>  Mus musculus

<400>  10

Trp Ile Asp Pro Glu Asn Gly Asn Thr Ile Tyr Asp Pro Lys Phe Gln
1               5                   10                  15

Asp

<210>  11
<211>  10
<212>  PRT
<213>  Mus musculus

<400>  11

Glu Arg Gly Tyr Asp Tyr Ala Met Asp Tyr
1               5                   10

<210>  12
<211>  11
<212>  PRT
<213>  Mus musculus

<400>  12

Arg Ala Ser Gln Ser Ile Ser Asn Asn Leu His
1               5                   10

<210>  13
<211>  7
<212>  PRT
<213>  Mus musculus

<400>  13

Tyr Ala Ser Gln Ser Ile Ser
1               5

<210>  14
<211>  10
<212>  PRT
<213>  Mus musculus

<400>  14

Gln Gln Ser Asn Ser Trp Pro Gln Tyr Thr
1               5                   10

<210>  15
<211>  116
<212>  PRT

<213>  Mus musculus

<400>  15

Glu Val Gln Leu Val Glu Ser Gly Gly Gly Leu Val Gln Pro Gly Lys
1               5                   10                  15

Ser Leu Lys Leu Ser Cys Glu Ala Ser Gly Phe Thr Phe Ser Gly Tyr
            20                  25                  30

Gly Met His Trp Val Arg Gln Ala Pro Gly Arg Gly Leu Glu Ser Val
        35                  40                  45

Ala Tyr Ile Thr Ser Ser Ser Ile Asn Ile Lys Tyr Ala Asp Ala Val
    50                  55                  60

Lys Gly Arg Phe Thr Val Ser Arg Asp Asn Ala Lys Asn Leu Leu Phe
65                  70                  75                  80

Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser
        115

<210>  16
<211>  107
<212>  PRT
<213>  Mus musculus

<400>  16

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80

Glu Asp Ile Gly Ser Tyr Tyr Cys Gln Gln Tyr Tyr Asn Tyr Pro Trp
                85                  90                  95

Thr Phe Gly Pro Gly Thr Lys Leu Glu Ile Lys
            100                 105

<210>  17
<211>  5
<212>  PRT
<213>  Mus musculus

<400>  17

Gly Tyr Gly Met His
1               5

<210>  18
<211>  17
<212>  PRT
<213>  Mus musculus

<400>  18

Tyr Ile Thr Ser Ser Ser Ile Asn Ile Lys Tyr Ala Asp Ala Val Lys
1               5                   10                  15

Gly

<210>  19
<211>  7
<212>  PRT
<213>  Mus musculus

<400>  19

Phe Asp Trp Asp Lys Asn Tyr
1               5

<210>  20
<211>  11
<212>  PRT
<213>  Mus musculus

<400>  20

Gln Ala Ser Gln Asp Ile Ser Asn Tyr Leu Asn
1               5                   10

<210>  21
<211>  7
<212>  PRT
<213>  Mus musculus

<400>  21

Tyr Thr Asn Lys Leu Ala Asp
1               5

<210>  22
<211>  9
<212>  PRT
<213>  Mus musculus

<400>  22

Gln Gln Tyr Tyr Asn Tyr Pro Trp Thr
1               5

<210>  23
<211>  124
<212>  PRT
<213>  Mus musculus

<400>  23

Glu Val Lys Leu Gln Glu Ser Gly Gly Gly Leu Val Gln Pro Gly Arg
1               5                   10                  15

Ser Leu Lys Leu Ser Cys Ala Ala Ser Gly Phe Asn Phe Asn Asp Tyr
            20                  25                  30

Trp Met Gly Trp Val Arg Gln Ala Pro Gly Lys Gly Leu Glu Trp Ile
        35                  40                  45

Gly Glu Ile Asn Lys Asp Ser Ser Thr Ile Asn Tyr Thr Pro Ser Leu
    50                  55                  60

Lys Asp Lys Phe Thr Ile Ser Arg Asp Asn Ala Gln Asn Thr Leu Tyr
65                  70                  75                  80

Leu Gln Met Ser Lys Leu Gly Ser Glu Asp Thr Ala Ile Tyr Tyr Cys
                85                  90                  95

Ala Arg Ala Arg Gly Met Met Val Leu Ile Ile Pro His Tyr Phe Asp
            100                 105                 110

Tyr Trp Gly Gln Gly Val Met Val Thr Val Ser Ser
        115                 120

<210>  24
<211>  106
<212>  PRT
<213>  Mus musculus

<400>  24

Asp Ile Val Leu Thr Gln Ser Pro Ala Met Ala Met Ser Pro Gly Glu
1               5                   10                  15

Arg Ile Thr Ile Ser Cys Arg Ala Ser Glu Ser Val Ser Thr Arg Met
            20                  25                  30

His Trp Tyr Gln Gln Lys Pro Gly Gln Gln Pro Lys Leu Leu Ile Tyr
        35                  40                  45

Gly Ala Ser Asn Leu Glu Ser Gly Val Pro Ala Arg Phe Ser Gly Ser
    50                  55                  60

Gly Ser Gly Thr Asp Phe Thr Leu Thr Ile Asp Pro Val Glu Ala Asn
65                  70                  75                  80

Asp Thr Ala Thr Tyr Phe Cys Gln Gln Ser Trp Tyr Asp Pro Trp Thr
                85                  90                  95

Phe Gly Gly Gly Thr Lys Leu Glu Leu Lys
            100                 105

<210>  25
<211>  5
<212>  PRT
<213>  Mus musculus

<400>  25

Asp Tyr Trp Met Gly
1               5

<210>  26
<211>  17
<212>  PRT
<213>  Mus musculus

<400>  26

Glu Ile Asn Lys Asp Ser Ser Thr Ile Asn Tyr Thr Pro Ser Leu Lys
1               5                   10                  15

Asp

<210>  27
<211>  15
<212>  PRT
<213>  Mus musculus

<400>  27

Ala Arg Gly Met Met Val Leu Ile Ile Pro His Tyr Phe Asp Tyr
1               5                   10                  15

<210>  28
<211>  11
<212>  PRT
<213>  Mus musculus

<400>  28

Arg Ala Ser Glu Ser Val Ser Thr Arg Met His
1               5                   10


<210>  29
<211>  7
<212>  PRT
<213>  Mus musculus

<400>  29

Gly Ala Ser Asn Leu Glu Ser
1               5


<210>  30
<211>  9
<212>  PRT
<213>  Mus musculus

<400>  30

Gln Gln Ser Trp Tyr Asp Pro Trp Thr
1               5


<210>  31
<211>  524
<212>  PRT
<213>  Artificial Sequence

<220>
<223>  Fusion protein

<400>  31

Met Glu Thr Asp Thr Leu Leu Leu Trp Val Leu Leu Leu Trp Val Pro
1               5                   10                  15


Gly Ser Thr Gly Asp Ala Ala Gln Pro Ala Arg Glu Val Gln Leu Val
            20                  25                  30


Glu Ser Gly Gly Gly Leu Val Gln Pro Gly Lys Ser Leu Lys Leu Ser
        35                  40                  45


Cys Glu Ala Ser Gly Phe Thr Phe Ser Gly Tyr Gly Met His Trp Val
    50                  55                  60


Arg Gln Ala Pro Gly Arg Gly Leu Glu Ser Val Ala Tyr Ile Thr Ser
65                  70                  75                  80

Ser Ser Ile Asn Ile Lys Tyr Ala Asp Ala Val Lys Gly Arg Phe Thr
                85                  90                  95

Val Ser Arg Asp Asn Ala Lys Asn Leu Leu Phe Leu Gln Met Asn Ile
            100                 105                 110

Leu Lys Ser Glu Asp Thr Ala Met Tyr Tyr Cys Ala Arg Phe Asp Trp
        115                 120                 125

Asp Lys Asn Tyr Trp Gly Gln Gly Thr Met Val Thr Val Ser Ser Gly
    130                 135                 140

Gly Gly Gly Ser Ile Val Leu Thr Gln Ser Pro Ala Thr Leu Ser Val
145                 150                 155                 160

Thr Pro Gly Asp Ser Val Ser Leu Ser Cys Arg Ala Ser Gln Ser Ile
                165                 170                 175

Ser Asn Asn Leu His Trp Tyr Gln Gln Lys Ser His Glu Ser Pro Arg
            180                 185                 190

Leu Leu Ile Lys Tyr Ala Ser Gln Ser Ile Ser Gly Ile Pro Ser Arg
        195                 200                 205

Phe Ser Gly Ser Gly Ser Gly Thr Asp Phe Thr Leu Ser Ile Asn Ser
    210                 215                 220

Val Glu Thr Glu Asp Phe Gly Met Tyr Phe Cys Gln Gln Ser Asn Ser
225                 230                 235                 240

Trp Pro Gln Tyr Thr Phe Gly Gly Gly Thr Lys Leu Glu Ile Lys Gly
                245                 250                 255

Gly Gly Gly Ser Gly Gly Gly Gly Ser Gly Gly Gly Gly Ser Val Gln
            260                 265                 270

Leu Gln Gln Ser Gly Ala Glu Leu Val Arg Pro Gly Ala Leu Val Lys
        275                 280                 285

Leu Ser Cys Lys Ala Ser Gly Phe Asn Ile Lys Asp Tyr Tyr Met His
    290                 295                 300

Trp Val Lys Gln Arg Pro Glu Gln Gly Leu Glu Trp Ile Gly Trp Ile
305                 310                 315                 320

Asp Pro Glu Asn Gly Asn Thr Ile Tyr Asp Pro Lys Phe Gln Asp Lys
                325                 330                 335

Ala Ser Leu Thr Ala Asp Thr Ser Ser Asn Thr Ala Tyr Leu Gln Leu
            340                 345                 350

Ser Ser Leu Thr Ser Glu Asp Thr Ala Val Tyr Tyr Cys Ala Pro Glu
        355                 360                 365

Arg Gly Tyr Asp Tyr Ala Met Asp Tyr Trp Gly Gln Gly Thr Ser Val
    370                 375                 380

Thr Val Ser Ser Gly Gly Gly Gly Ser Asp Ile Gln Met Thr Gln Ser
385                 390                 395                 400

Pro Ser Ser Leu Pro Ala Ser Leu Gly Asp Arg Val Thr Ile Asn Cys
                405                 410                 415

Gln Ala Ser Gln Asp Ile Ser Asn Tyr Leu Asn Trp Tyr Gln Gln Lys
            420                 425                 430

Pro Gly Lys Ala Pro Lys Leu Leu Ile Tyr Tyr Thr Asn Lys Leu Ala
        435                 440                 445

Asp Gly Val Pro Ser Arg Phe Ser Gly Ser Gly Ser Gly Arg Asp Ser
    450                 455                 460

Ser Phe Thr Ile Ser Ser Leu Glu Ser Glu Asp Ile Gly Ser Tyr Tyr
465                 470                 475                 480

Cys Gln Gln Tyr Tyr Asn Tyr Pro Trp Thr Phe Gly Pro Gly Thr Lys
                485                 490                 495

Leu Glu Ile Lys Gly Gly Gly Gly Ser Asp Tyr Lys Asp Asp Asp Asp
            500                 505                 510

Lys Asn Ser Ala Val Asp His His His His His His
        515                 520

<210>  32
<211>  524
<212>  PRT
<213>  Artificial Sequence

<220>
<223>  Fusion protein

<400>  32

Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Val Gln Leu Gln Gln
            20                  25                  30

Ser Gly Ala Glu Leu Val Arg Pro Gly Ala Leu Val Lys Leu Ser Cys
        35                  40                  45

Lys Ala Ser Gly Phe Asn Ile Lys Asp Tyr Tyr Met His Trp Val Lys
    50                  55                  60

Gln Arg Pro Glu Gln Gly Leu Glu Trp Ile Gly Trp Ile Asp Pro Glu
65                  70                  75                  80

Asn Gly Asn Thr Ile Tyr Asp Pro Lys Phe Gln Asp Lys Ala Ser Leu
                85                  90                  95

Thr Ala Asp Thr Ser Ser Asn Thr Ala Tyr Leu Gln Leu Ser Ser Leu
            100                 105                 110

Thr Ser Glu Asp Thr Ala Val Tyr Tyr Cys Ala Pro Glu Arg Gly Tyr
        115                 120                 125

Asp Tyr Ala Met Asp Tyr Trp Gly Gln Gly Thr Ser Val Thr Val Ser
    130                 135                 140

Ser Gly Gly Gly Gly Ser Asp Ile Gln Met Thr Gln Ser Pro Ser Ser
145                 150                 155                 160

Leu Pro Ala Ser Leu Gly Asp Arg Val Thr Ile Asn Cys Gln Ala Ser
                165                 170                 175

Gln Asp Ile Ser Asn Tyr Leu Asn Trp Tyr Gln Gln Lys Pro Gly Lys
            180                 185                 190

Ala Pro Lys Leu Leu Ile Tyr Tyr Thr Asn Lys Leu Ala Asp Gly Val
        195                 200                 205

Pro Ser Arg Phe Ser Gly Ser Gly Ser Gly Arg Asp Ser Ser Phe Thr
    210                 215                 220

Ile Ser Ser Leu Glu Ser Glu Asp Ile Gly Ser Tyr Tyr Cys Gln Gln
225                 230                 235                 240

Tyr Tyr Asn Tyr Pro Trp Thr Phe Gly Pro Gly Thr Lys Leu Glu Ile
                245                 250                 255

Lys Gly Gly Gly Gly Ser Gly Gly Gly Gly Ser Gly Gly Gly Gly Ser
            260                 265                 270

Glu Val Gln Leu Val Glu Ser Gly Gly Gly Leu Val Gln Pro Gly Lys
        275                 280                 285

Ser Leu Lys Leu Ser Cys Glu Ala Ser Gly Phe Thr Phe Ser Gly Tyr
    290                 295                 300

Gly Met His Trp Val Arg Gln Ala Pro Gly Arg Gly Leu Glu Ser Val
305                 310                 315                 320

Ala Tyr Ile Thr Ser Ser Ser Ile Asn Ile Lys Tyr Ala Asp Ala Val
                325                 330                 335

Lys Gly Arg Phe Thr Val Ser Arg Asp Asn Ala Lys Asn Leu Leu Phe
            340                 345                 350

Leu Gln Met Asn Ile Leu Lys Ser Glu Asp Thr Ala Met Tyr Tyr Cys
        355                 360                 365

Ala Arg Phe Asp Trp Asp Lys Asn Tyr Trp Gly Gln Gly Thr Met Val
    370                 375                 380

Thr Val Ser Ser Gly Gly Gly Gly Ser Ile Val Leu Thr Gln Ser Pro
385                 390                 395                 400

Ala Thr Leu Ser Val Thr Pro Gly Asp Ser Val Ser Leu Ser Cys Arg
                405                 410                 415

Ala Ser Gln Ser Ile Ser Asn Asn Leu His Trp Tyr Gln Gln Lys Ser
            420                 425                 430

His Glu Ser Pro Arg Leu Leu Ile Lys Tyr Ala Ser Gln Ser Ile Ser
        435                 440                 445

Gly Ile Pro Ser Arg Phe Ser Gly Ser Gly Ser Gly Thr Asp Phe Thr
    450                 455                 460

Leu Ser Ile Asn Ser Val Glu Thr Glu Asp Phe Gly Met Tyr Phe Cys
465                 470                 475                 480

Gln Gln Ser Asn Ser Trp Pro Gln Tyr Thr Phe Gly Gly Gly Thr Lys
                485                 490                 495

Leu Glu Ile Lys Gly Gly Gly Gly Ser Asp Tyr Lys Asp Asp Asp Asp
            500                 505                 510

Lys Asn Ser Ala Val Asp His His His His His His
        515                 520

<210>  33
<211>  532
<212>  PRT
<213>  Artificial Sequence

<220>
<223>  Fusion protein

<400>  33

Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Val Gln Leu Gln Gln
            20                  25                  30

Ser Gly Ala Glu Leu Val Arg Pro Gly Ala Leu Val Lys Leu Ser Cys
        35                  40                  45

Lys Ala Ser Gly Phe Asn Ile Lys Asp Tyr Tyr Met His Trp Val Lys
    50                  55                  60

Gln Arg Pro Glu Gln Gly Leu Glu Trp Ile Gly Trp Ile Asp Pro Glu
65                  70                  75                  80

Asn Gly Asn Thr Ile Tyr Asp Pro Lys Phe Gln Asp Lys Ala Ser Leu
                85                  90                  95

Thr Ala Asp Thr Ser Ser Asn Thr Ala Tyr Leu Gln Leu Ser Ser Leu
            100                 105                 110

Thr Ser Glu Asp Thr Ala Val Tyr Tyr Cys Ala Pro Glu Arg Gly Tyr
        115                 120                 125

Asp Tyr Ala Met Asp Tyr Trp Gly Gln Gly Thr Ser Val Thr Val Ser
    130                 135                 140

Ser Gly Gly Gly Gly Ser Asp Ile Val Leu Thr Gln Ser Pro Ala Met
145                 150                 155                 160

Ala Met Ser Pro Gly Glu Arg Ile Thr Ile Ser Cys Arg Ala Ser Glu
                165                 170                 175

Ser Val Ser Thr Arg Met His Trp Tyr Gln Gln Lys Pro Gly Gln Gln
            180                 185                 190

Pro Lys Leu Leu Ile Tyr Gly Ala Ser Asn Leu Glu Ser Gly Val Pro
        195                 200                 205

Ala Arg Phe Ser Gly Ser Gly Ser Gly Thr Asp Phe Thr Leu Thr Ile
    210                 215                 220

Asp Pro Val Glu Ala Asn Asp Thr Ala Thr Tyr Phe Cys Gln Gln Ser
225                 230                 235                 240

Trp Tyr Asp Pro Trp Thr Phe Gly Gly Gly Thr Lys Leu Glu Leu Lys
                245                 250                 255

Gly Gly Gly Gly Ser Gly Gly Gly Gly Ser Gly Gly Gly Gly Ser Glu
            260                 265                 270

Val Lys Leu Gln Glu Ser Gly Gly Gly Leu Val Gln Pro Gly Arg Ser
        275                 280                 285

Leu Lys Leu Ser Cys Ala Ala Ser Gly Phe Asn Phe Asn Asp Tyr Trp
    290                 295                 300

Met Gly Trp Val Arg Gln Ala Pro Gly Lys Gly Leu Glu Trp Ile Gly
305                 310                 315                 320

Glu Ile Asn Lys Asp Ser Ser Thr Ile Asn Tyr Thr Pro Ser Leu Lys
                325                 330                 335

Asp Lys Phe Thr Ile Ser Arg Asp Asn Ala Gln Asn Thr Leu Tyr Leu
            340                 345                 350

Gln Met Ser Lys Leu Gly Ser Glu Asp Thr Ala Ile Tyr Tyr Cys Ala
        355                 360                 365

Arg Ala Arg Gly Met Met Val Leu Ile Ile Pro His Tyr Phe Asp Tyr
    370                 375                 380

Trp Gly Gln Gly Val Met Val Thr Val Ser Ser Thr Gly Gly Gly Gly
385                 390                 395                 400

Ser Ile Val Leu Thr Gln Ser Pro Ala Thr Leu Ser Val Thr Pro Gly
                405                 410                 415

Asp Ser Val Ser Leu Ser Cys Arg Ala Ser Gln Ser Ile Ser Asn Asn
            420                 425                 430

Leu His Trp Tyr Gln Gln Lys Ser His Glu Ser Pro Arg Leu Leu Ile
        435                 440                 445

Lys Tyr Ala Ser Gln Ser Ile Ser Gly Ile Pro Ser Arg Phe Ser Gly
    450                 455                 460

Ser Gly Ser Gly Thr Asp Phe Thr Leu Ser Ile Asn Ser Val Glu Thr
465                 470                 475                 480

Glu Asp Phe Gly Met Tyr Phe Cys Gln Gln Ser Asn Ser Trp Pro Gln
                485                 490                 495

Tyr Thr Phe Gly Gly Gly Thr Lys Leu Glu Ile Lys Gly Gly Gly Gly
            500                 505                 510

Ser Asp Tyr Lys Asp Asp Asp Asp Lys Asn Ser Ala Val Asp His His
        515                 520                 525

His His His His
    530

<210>  34
<211>  25
<212>  PRT
<213>  Artificial Sequence

<220>
<223>  linker

<400>  34

Ser Ser Ala Asp Asp Ala Lys Lys Asp Ala Ala Lys Lys Asp Asp Ala
1               5                   10                  15

Lys Lys Asp Asp Ala Lys Lys Asp Ala
            20                  25

## Description

### TECHNICAL FIELD

This patent application claims the benefit of priority of Japanese Patent Application No. 2019-108906, the entire content of which is incorporated herein by reference.

The present disclosure relates to a bispecific molecule including a first binding site that specifically binds to LAG3 and a second binding site that specifically binds to CD3 or CD8.

### BACKGROUND ART

The immune system is a mechanism in which multiple mechanisms are integrated, the mechanism protecting the living body from disease by recognizing and killing non-self substances such as pathogens and abnormal cells such as cancer cells in the body. The immune system is tightly regulated so that it attacks pathogens and abnormal cells and does not attack normal self-substances, but if the control mechanism is disrupted, various intractable diseases such as autoimmune diseases and chronic inflammatory diseases are caused. Regarding the treatment of autoimmune diseases, use of immunosuppressants and antibody therapy targeting cytokines are known.

A lymphocyte activation gene 3 (LAG3) is an immune checkpoint receptor protein expressed on the surface of a cytotoxic T cell and a regulatory T cell, and regulates T cell response, activation, and proliferation by suppressing a T cell receptor (TCR). A method for activating LAG3 has not been known so far. In general, inhibition of the function of a cell surface molecule can be achieved by inhibiting the binding of a ligand to a cell surface molecule physically with an antibody, but activation of the function of a cell surface molecule is extremely difficult because it requires specific structural changes to be induced by a ligand or the like.

CD3 is mainly expressed in a mature T cell and binds to the TCR to form a complex. When a foreign antigen is presented to the TCR via an MHC complex and the activation of T cells is induced, CD3 undertakes a function of intracellular signaling. CD8 is a co-receptor of the TCR expressed on the surface of a T cell, and binds to a conserved region of the MHC class I molecule. When a specific antigen is presented to mature naive CD8⁺T cells by dendritic cells, CD8 and TCR assemble, intracellular signaling is initiated, and the mature naive CD8⁺T cells differentiate into cytotoxic T cells. That is, both CD3 and CD8 are cell surface molecules that assemble with the TCR upon T cell activation.

Patent Literature 1 discloses that an immune response suppressing activity of a cell having a function of suppressing an immune response, such as a regulatory T cell, is inhibited by an antigen binding molecule including a domain that binds to a molecule expressed on a surface of a cell having a function of suppressing an immune response and a domain binding to a T cell receptor complex.

### CITATIONS LIST

### Patent Literature

Patent Literature 1: WO2015/174439

### SUMMARY OF INVENTION

### TECHNICAL PROBLEMS

It is an object of the present disclosure to provide a new immunosuppressant.

### SOLUTIONS TO PROBLEMS

In a certain aspect, the present disclosure provides a bispecific molecule including a first binding site that specifically binds to LAG3 and a second binding site that specifically binds to CD3 or CD8.

In a certain aspect, the present disclosure provides an immunosuppressant containing the above-described bispecific molecule.

In one aspect, the present disclosure provides a prophylactic and/or therapeutic agent for an autoimmune disease, an allergic disease, or a graft-versus-host disease, containing the above-described bispecific molecule.

### ADVANTAGEOUS EFFECTS OF INVENTION

The present disclosure provides: immunosuppressants; prophylactic and/or therapeutic agents for autoimmune diseases, allergic diseases, or graft-versus-host diseases; or bispecific molecules that can be utilized for these.

### BRIEF DESCRIPTION OF DRAWINGS

Fig. 1 shows binding to BW5147 cells, DO11.10 cells, and DO11.10-mLAG3 cells of bispecific molecules 2C11xTKB58 and TKB58x2C11 that bind to LAG3 and CD3.
Fig. 2 shows IL-2 production by DO 11.10 cells and DO 11.10-mLAG3 cells by antigen stimulation under conditions that strongly induce suppression by LAG3, in the presence of an anti-mouse LAG3 antibody TKB58, 2C11xTKB58, or TKB58x2C11.
Fig. 3 shows IL-2 production by DO11.10 cells and D011.10-mLAG3 cells by antigen stimulation under conditions that induce suppression by LAG3 less strongly, in the presence of TKB58, 2C11xTKB58 or TKB58x2C11.
Fig. 4 shows IL-2 production by MHC class I-restricted B3Z cells and B3Z-mLAG3 cells by antigen stimulation, in the presence of TKB58, 2C11xTKB58 or TKB58x2C11.
Fig. 5 shows IL-2 production by DO11.10 cells and DO11.10-mLAG3-P111A cells by antigen stimulation, in the presence of TKB58, 2C11xTKB58 or TKB58x2C11.
Fig. 6 shows the effect of 2C11xTKB58 on neurological symptoms in an experimental autoimmune encephalomyelitis (EAE) model.
Fig. 7 shows binding of an LAG3 soluble protein to ILA1.6 cells treated with TKB58, 2C11xTKB58 or TKB58x2C11.
Fig. 8 shows binding of a bispecific molecule TKB58xYTS169 to DO11.10 cells, DO11.10-mLAG3 cells, B3Z cells, and B3Z-mLAG3 cells.
Fig. 9 shows IL-2 production of B3Z cells and B3Z-mLAG3 cells by antigen stimulation, in the presence of TKB58xYTS169.
Fig. 10 shows binding of TKB58 to DO11.10 cells expressing a chimeric LAG3 molecule.
Fig. 11 shows binding of TKB58 to DO11.10 cells in which wild-type LAG3, an N54A/F55A mutant, or a V61A/I62A mutant is expressed.
Fig. 12 shows IL-2 production by antigen stimulation in DO11.10 cells in which wild-type LAG3, an N54A/F55A mutant, or a V61A/I62A mutant is expressed.
Fig. 13 shows the binding affinity of anti-mouse LAG3 antibodies TKB58 and C9B7W to a mouse LAG3 soluble protein.

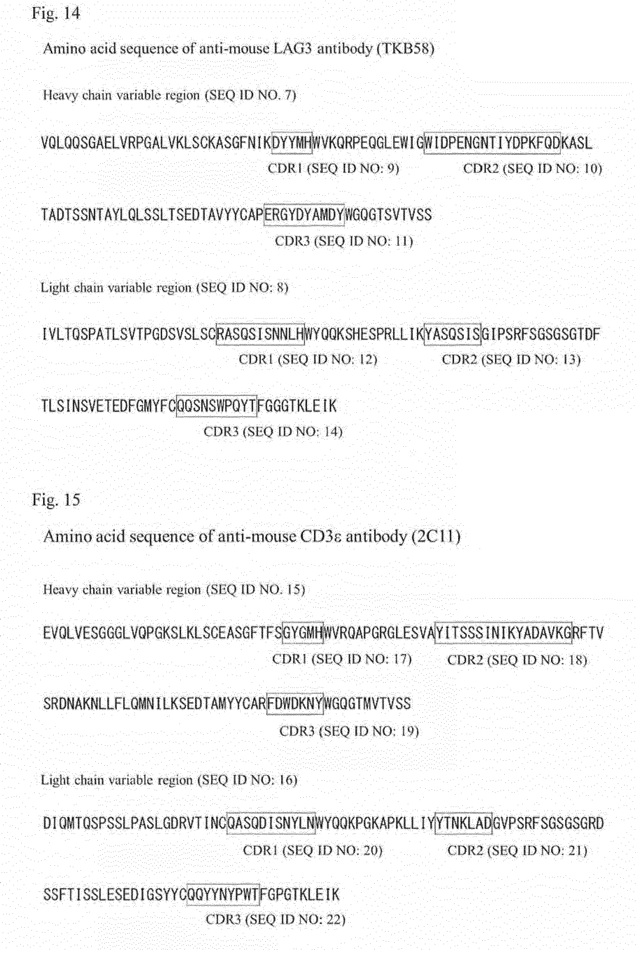

Fig. 14 shows amino acid sequences of heavy and light chain variable regions of an anti-mouse LAG3 antibody TKB58. Each CDR is shown in a square.
Fig. 15 shows amino acid sequences of heavy and light chain variable regions of an anti-mouse CD3ε antibody 2C11. Each CDR is shown in a square.

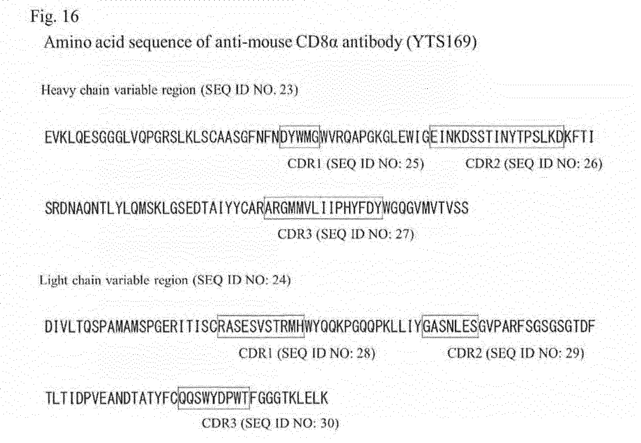

Fig. 16 shows amino acid sequences of heavy and light chain variable regions of an anti-mouse CD8 antibody YTS169. Each CDR is shown in a square.

### DESCRIPTION OF EMBODIMENTS

In the present disclosure, when a numerical value is accompanied by the term "about", it is intended to encompass a range of ± 10% of that value. For example, "about 20" shall encompass "18-22". A range expressed by numerical values encompasses all values between these numerical values, and the numerical values at both of the ends. The "about" for a range applies to both ends of the range. Therefore, for example, "about 20-30" encompasses "18-33".

In the present disclosure, amino acid residues are represented by the following abbreviations.
Ala or A: alanine
Arg or R: arginine
Asn or N: asparagine
Asp or D: aspartic acid
Cys or C: cysteine
Gln or Q: glutamine
Glu or E: glutamic acid
Gly or G: glycine
His or H: histidine
Ile or I: isoleucine
Leu or L: leucine
Lys or K: lysine
Met or M: methionine
Phe or F: phenylalanine
Pro or P: proline
Ser or S: serine
Thr or T: threonine
Trp or W: tryptophan
Tyr or Y: tyrosine
Val or V: valine

By "bispecific molecule" is meant a molecule capable of specifically binding to two different target molecules or target sites. The bispecific molecule includes: a first binding site that specifically binds to a first target molecule or site; and a second binding site that specifically binds to a second target molecule or site. Here, the "first binding site" and the "second binding site" are terms used for convenience to distinguish between two binding sites, and do not specify the position or function of the binding site in the bispecific molecule. A bispecific molecule may be composed of one molecule (e.g., a polypeptide chain) or may be composed of a plurality of molecules (e.g., a plurality of polypeptide chains). The bispecific molecule may be a multispecific molecule having at least one further binding site, wherein the further binding site may be a site that is the same as or different from the first or second binding site, may be a site that specifically binds to a first or second target molecule or site, or may be a site that specifically binds to a target molecule or target site different from the first or second target molecule or site.

In the present disclosure, LAG3, CD3 and CD8 may be of any species, and are typically mammalian (for example, human, mouse, rat, hamster, rabbit, cat, dog, cow, sheep, monkey, and the like), for example, mouse or human, particularly human. The amino acid sequences of LAG3, CD3, and CD8 derived from various species are readily available using known databases. In the present disclosure, LAG3, CD3, and CD8 encompass the products of their naturally occurring alleles.

LAG3 selectively binds to a stable peptide MHCII complex and suppresses a T-cell receptor (TCR), thereby suppressing T-cell response, activation and/or proliferation. Representative amino acid sequences of human and mouse LAG3 are registered as GenBank accession numbers NP_002277.4 (SEQ ID NO: 1) and NP_0S2505.1 (SEQ ID NO: 2), respectively.

The first binding site may bind anywhere in the extracellular region of LAG3. In a certain embodiment, the first binding site binds to a D1 region of LAG3, particularly to a portion that is included in the D1 region and not included in an extra loop region. The D1 region and the extra loop region are described in PNAS, 1997, 94 (11): 5744-5749, Journal of Immunology, 1996, 157: 3727-3736, and the like. For example, in mouse LAG3 having the amino acid sequence of SEQ ID NO: 2, the D1 region is a region ranging from serine at position 23 to isoleucine at position 168, and the extra loop region is a region ranging from glycine at position 70 to tyrosine at position 95. Preferably, the first binding site binds to a region on an N-terminal side with respect to the extra loop region in the D1 region (a region ranging from serine at position 23 to serine at position 69 in SEQ ID NO: 2). In one embodiment, the first binding site binds to a region corresponding to a region including serine at position 23 to serine at position 69 of mouse LAG3 having the amino acid sequence of SEQ ID NO: 2. For example, the region including leucine at position 23 to serine at position 69 of human LAG3 having the amino acid sequence of SEQ ID NO: 1 corresponds to the region ranging from serine at position 23 to serine at position 69 of mouse LAG3 having the amino acid sequence of SEQ ID NO: 2. In a certain embodiment, the first binding site binds to a region including an amino acid corresponding to asparagine at position 54, phenylalanine at position 55, valine at position 61, and/or isoleucine at position 62 of mouse LAG3 having the amino acid sequence of SEQ ID NO: 2. For example, a region including serine at position 54, leucine at position 55, valine at position 61, and/or threonine at position 62 of human LAG3 having the amino acid sequence of SEQ ID NO: 1 corresponds to a region including asparagine at position 54, phenylalanine at position 55, valine at position 61, and/or isoleucine at position 62 of mouse LAG3 having the amino acid sequence of SEQ ID NO: 2.

Preferably, the bispecific molecule allows binding of LAG3 to MHC class II molecules. By "allowing binding" is meant that the amount of binding between LAG3 and MHC class II molecules is not substantially reduced in the presence of the bispecific molecule, for example, it is greater than or equal to about 70%, about 75%, about 80%, about 85%, about 90%, about 95%, or about 100% of the amount of binding in the absence of the bispecific molecule. Binding of LAG3 to MHC class II molecules can be confirmed by experiments in which an extracellular region of LAG-3 as a soluble protein is bound to cells expressing MHC class II in the presence and absence of bispecific molecules. Alternatively, the binding of LAG3 to MHC class II molecules can be confirmed by comparing amounts of cytokine production in the presence and absence of the bispecific molecule, for example, in a system in which LAG3, binding to the peptide MHCII complex, suppresses cytokine production by TCR, for example, a system described in the Examples below.

CD3 is mainly expressed in mature T cells and forms a complex with the TCR. CD3 includes subunits of CD3ζ, CD3ε, CD3γ, CD3δ, and CD3η. The second binding site may bind to any subunit. In certain embodiments, the second binding site of the bispecific molecule binds to CD3ε. Representative amino acid sequences of human and mouse CD3ε are registered as GenBank accession numbers NP_000724.1 (SEQ ID NO: 3) and NP031674.1 (SEQ ID NO: 4), respectively. The second binding site may bind anywhere in the extracellular region of CD3. In a certain embodiment, the second binding site binds to an extracellular region of CD3ε. For example, in human CD3ε having the amino acid sequence of SEQ ID NO: 3, the extracellular region is a region ranging from aspartic acid at position 23 to aspartic acid at position 126, and in mouse CD3ε having the amino acid sequence of SEQ ID NO: 4, the extracellular region is a region ranging from aspartic acid at position 22 to aspartic acid at position 108. In a certain embodiment, the second binding site binds to a region including amino acids corresponding to aspartic acid at position 22 to asparagine at position 26, leucine at position 44 to asparagine at position 49, lysine at position 51, and/or tyrosine at position 84 to asparagine at position 91 of mouse CD3ε having the amino acid sequence of SEQ ID NO: 4.

CD8 is a co-receptor of the TCR expressed on the surface of T cells, and when mature naïve CD8+T cells are presented with specific antigens by dendritic cells, CD8 and TCR assemble. CD8 includes subunits of CD8α and CD8β. The second binding site may bind to any subunit. In a certain embodiment, the second binding site of the bispecific molecule binds to CD8α. Representative amino acid sequences of human and mouse CD8α are registered as GenBank accession numbers NP_001139345.1 (SEQ ID NO: 5) and NP_001074579.1 (SEQ ID NO: 6), respectively. The second binding site may bind to anywhere in the extracellular region of CD8. In a certain embodiment, the second binding site binds to an extracellular region of CD8α. For example, in human CD8α having the amino acid sequence of SEQ ID NO: 5, the extracellular region is a region ranging from serine at position 22 to aspartic acid at position 182, and in mouse CD8α having the amino acid sequence of SEQ ID NO: 6, the extracellular region is a region ranging from lysine at position 28 to tyrosine at position 196.

"An amino acid corresponding to asparagine at position 54 of mouse LAG3 having the amino acid sequence of SEQ ID NO: 2" means an amino acid in the LAG3 that matches with asparagine at position 54 of SEQ ID NO: 2 when a certain amino acid sequence of LAG3 and the amino acid sequence of SEQ ID NO: 2 are aligned in an optimum state (the state in which the amino acid matching is maximized). The sequences of CD3 and CD8 are similarly defined. For example, serine at position 54, leucine at position 55, valine at position 61, and threonine at position 62 in human LAG3 having the amino acid sequence of SEQ ID NO: 1 correspond to asparagine at position 54, phenylalanine at position 55, valine at position 61, and isoleucine at position 62 in mouse LAG3 having the amino acid sequence of SEQ ID NO: 2, respectively. Aspartic acid at position 23 to glutamic acid at position 27, glutamine at position 51 to glutamic acid at position 56, leucine at position 58, and/or tyrosine at position 99 to asparagine at position 109 of human CD3ε having the amino acid sequence of SEQ ID NO: 3 correspond to aspartic acid at position 22 to asparagine at position 26, leucine at position 44 to asparagine at position 49, lysine at position 51, and/or tyrosine at position 84 to asparagine at position 91 of mouse CD3ε having the amino acid sequence of SEQ ID NO: 4.

The first and second binding sites may be derived from an antibody, in particular from an antigen-binding site (variable region) of the antibody. Typically, the first binding site is derived from heavy and light chain variable regions of an anti-LAG3 antibody, and the second binding site is derived from heavy and light chain variable regions of an anti-CD3 or anti-CD8 antibody.

The variable region may be derived from an antibody of any animal species. Examples thereof include mouse-, rat-, rabbit-, goat-, and human-derived antibodies, as well as humanized antibodies. The variable region may be derived from any immunoglobulin class of antibodies. Examples of the immunoglobulin class include IgA, IgD, IgE, IgG, and IgM, and examples of the subclass (isotype) of the immunoglobulin class include IgG1, IgG2, IgG3, IgG4, IgA1, and IgA2. In a certain embodiment, the variable region is of an IgG subclass, e.g., an IgG1 or IgG4 subclass.

The variable region may be derived from a monoclonal antibody. Hybridomas that secrete monoclonal antibodies can be produced according to a known method, for example, the method described in Kohler et al., Nature 256: 495, 1975. First, the immunogen is mixed with a suitable substance for enhancing antigenicity (e.g., keyhole limpet hemocyanin, bovine serum albumin, etc.) and, if necessary, an immunostimulant (such as Freund's complete or incomplete adjuvant), and non-human mammals such as rats, mice, rabbits, goats, or horses are immunized with the same. Usually, immunized animals are subjected to multiple rounds of immunization at intervals of 3 to 10 days, and 1 to 100 µg of the immunogenic peptide is administered. Immunocompetent cells (cells capable of producing antibodies in immune animals) are then collected from the immunized animals that have undergone multiple rounds of immunization, and are fused with myeloma cells that are not capable of producing autoantibodies (e.g., cells derived from mammals such as mice, rats, guinea pigs, hamsters, rabbits, or humans). For the cell fusion, the polyethylene glycol method, the electric fusion method, or the like is used. Furthermore, cells that have been successfully fused are selected based on selection markers that the fused cells have, and the reactivity of the antibody produced by the selected cells to the immunogen is confirmed by ELISA, radioimmunoassay, fluorescent antibody method, etc. Thereby, a hybridoma that produces the desired monoclonal antibody is obtained. The monoclonal antibody can be isolated from the culture supernatant in which the obtained hybridoma is cultured in vitro. It can also be cultured in vivo in ascites of mice, rats, guinea pigs, hamsters, rabbits, or the like and isolated from the ascites.

As an immunogen for obtaining an anti-LAG3 antibody, a peptide including an amino acid sequence of all or part of the extracellular region of LAG3, for example, about 5 to 50 amino acids, about 6 to 40 amino acids, about 7 to 35 amino acids, about 8 to 30 amino acids, about 9 to 25 amino acids, or about 10 to 20 amino acids of the amino acid sequence of the extracellular region of LAG3, may be used. For example, a peptide including a portion of the D1 region of LAG3, in particular a peptide including a portion included in the D1 region and not included in the extra loop region, may be used. Alternatively, for example, a peptide including a region including serine at position 54, leucine at position 55, valine at position 61 and/or threonine at position 62 of human LAG3 having the amino acid sequence of SEQ ID NO: 1, or a region including asparagine at position 54, phenylalanine at position 55, valine at position 61 and/or isoleucine at position 62 of mouse LAG3 having the amino acid sequence of SEQ ID NO: 2, may be used.

As an immunogen for obtaining an anti-CD3 antibody, a peptide including an amino acid sequence of all or part of the extracellular region of CD3ε, for example, about 5 to 50 amino acids, about 6 to 40 amino acids, about 7 to 35 amino acids, about 8 to 30 amino acids, about 9 to 25 amino acids, or about 10 to 20 amino acids of the amino acid sequence of the extracellular region of CD3ε; may be used. For example, a peptide including a region including aspartic acid at position 23 to glutamic acid at position 27, glutamine at position 51 to glutamic acid at position 56, leucine at position 58, and/or tyrosine at position 99 to asparagine at position 109 of human CD3ε having the amino acid sequence of SEQ ID NO: 3, or a peptide including a region including aspartic acid at position 22 to asparagine at position 26, leucine at position 44 to asparagine at position 49, lysine at position 51, and/or tyrosine at position 84 to asparagine at position 91 of mouse CD3ε having the amino acid sequence of SEQ ID NO: 4 may be used.

As an immunogen for obtaining an anti-CD8 antibody, a peptide including an amino acid sequence of all or part of the extracellular region of CD8α, for example, about 5 to 50 amino acids, about 6 to 40 amino acids, about 7 to 35 amino acids, about 8 to 30 amino acids, about 9 to 25 amino acids, or about 10 to 20 amino acids of the amino acid sequence of the extracellular region of CD8α, may be used.

For example, monoclonal antibodies are used that bind to an antigen with an equilibrium dissociation constant (Kd) of 10⁻⁸ M or less, such as 10⁻⁸ M to 10⁻¹⁵ M, 10⁻⁸ M to 10⁻¹³ M, 10⁻⁹ M to 10⁻¹² M, or 10⁻⁹ M to 10⁻¹¹ M. The binding of the antibody to the antigen can be confirmed by ELISA, the fluorescent antibody method, the radioimmunoassay (RIA), the BIACORE (registered trademark) surface plasmon resonance assay, etc. Binding of an antibody to an antigen can also be confirmed by the competition assay. For example, whether or not an antibody competes with a known antibody in binding to an antigen can be confirmed by FACS, ELISA, or the like. As a known anti-LAG3 antibody, a known anti-CD3 antibody, and a known anti-CD8 antibody, for example, an antibody can be used that has a heavy chain variable region described below, a light chain variable region described below, or a CDR sequence described below.

The antibody gene may be cloned from the hybridoma producing the desired antibody by well-known methods to determine the amino acid sequence of the variable region or the nucleic acid sequence encoding the same. An amino acid sequence of a known anti-LAG3 antibody, anti-CD3 antibody, or anti-CD8 antibody, or alternatively, a nucleic acid sequence encoding the same, may be utilized. The variable region is usually composed of three complementarity determining regions (also referred to as CDRs) interposed between four framework regions (also referred to as FRs). In this disclosure, the amino acid positions assigned to the CDR of the variable region of the antibody and the framework are defined according to Kabat (see Sequences of Proteins of Immunological Interest (National Institute of Health, Bethesda, Md., (1987) and (1991)).

The CDR is a region that substantially determines the binding specificity of an antibody, and shows great diversity in amino acid sequence. On the other hand, the amino acid sequences constituting an FR show high homology, even between antibodies having different binding specificities. Therefore, the binding specificity of one antibody can be transplanted to another antibody by CDR transplantation. For example, by transplanting the CDRs of an antibody derived from an animal other than human into a human antibody, a humanized antibody composed of the CDRs of the antibody derived from the animal other than human, the FRs derived from the human antibody, and the constant regions derived from the human antibody can be obtained. A humanized antibody can be prepared by a variety of methods, one example of which is Overlap Extension PCR (Almagro and Fransson, Front. Biosci. 13: 1619-1633 (2008)). A method for selecting an FR suitable for producing a humanized antibody is known. For example, an FR selected by the best fit method (Sims et al. J. Immunol. 151: 2296 (1993)), or an FR derived from a consensus sequence of a particular subgroup of a light chain or heavy chain variable region of a human antibody (Carter et al. Proc. Natl. Acad. Sci. USA 89: 4285 (1992); Presta et al. J. Immunol. 151: 2623(1993)) can be used. The variable regions of the humanized antibody thus obtained may be used.

Variable regions of a human antibody may be used. Human antibodies can be obtained, for example, by sensitizing human lymphocytes in vitro with desired antigens and then fusing the sensitized lymphocytes with human myeloma cells (Japanese Patent application Publication H1-59878). For human myeloma cells, which are fusion partners, for example, U266 can be used. Human antibodies can also be obtained by immunizing transgenic animals with the entire repertoire of human antibody genes with the desired antigen (Lonberg, Nat. Biotech. 23: 1117-1125, 2005). Furthermore, a technique for obtaining human antibodies by panning using a human antibody library is also known (Antibody Phage Display: Methods and Protocols, Methods in Molecular Biology 178, 2001). For example, a variable region of a human antibody is expressed as a single-chain antibody (scFv) on the surface of a phage by a phage display method, a phage that binds to an antigen is selected, and the gene of the selected phage is analyzed. This enables to determine a nuclear acid sequence encoding the variable region of the human antibody binding to the antigen.

In a certain embodiment, the first binding site that binds to LAG3 includes:
a heavy chain variable region including CDR1, CDR2 and CDR3 having the same amino acid sequences as those of CDR1, CDR2 and CDR3, respectively, included in a heavy chain variable region having the amino acid sequence of SEQ ID NO: 7; and/or
a light chain variable region including CDR1, CDR2 and CDR3 having the same amino acid sequences as those of CDR1, CDR2 and CDR3, respectively, included in a light chain variable region having the amino acid sequence of SEQ ID NO: 8.

In a certain embodiment, the first binding site that binds to LAG3 includes:
a heavy chain variable region including a heavy chain CDR1 including the amino acid sequence of SEQ ID NO: 9, a heavy chain CDR2 including the amino acid sequence of SEQ ID NO: 10, and a heavy chain CDR3 including the amino acid sequence of SEQ ID NO: 11; and/or
a light chain variable region including a light chain CDR1 including the amino acid sequence of SEQ ID NO: 12, a light chain CDR2 including the amino acid sequence of SEQ ID NO: 13, and a light chain CDR3 including the amino acid sequence of SEQ ID NO: 14.

In a certain embodiment, the first binding site that binds to LAG3 includes:
a heavy chain variable region that includes: a heavy chain CDR1 consisting of the amino acid sequence of SEQ ID NO: 9, a heavy chain CDR2 consisting of an amino acid sequence of SEQ ID NO: 10, and heavy chain CDR3 consisting of the amino acid sequence of SEQ ID NO: 11; and/or
a light chain variable region that includes a light chain CDR1 consisting of the amino acid sequence of SEQ ID NO: 12, a light chain CDR2 consisting of the amino acid sequence of SEQ ID NO: 13, and a light chain CDR3 consisting of the amino acid sequence of SEQ ID NO: 14.

In a certain embodiment, the first binding site that binds to LAG3 includes:
a heavy chain variable region including:
a CDR1 including a sequence having a sequence identity of 80% or more, preferably 85% or more, more preferably 90% or more, and even more preferably 95% or more with the sequence of SEQ ID NO: 9;
a CDR2 including a sequence having a sequence identity of 80% or more, preferably 85% or more, more preferably 90% or more, and even more preferably 95% or more with the sequence of SEQ ID NO: 10; and
a CDR3 including a sequence having a sequence identity of 80% or more, preferably 85% or more, more preferably 90% or more, and even more preferably 95% or more with the sequence of SEQ ID NO: 11; and/or
a light chain variable region including:
   a CDR1 including a sequence having a sequence identity of 80% or more, preferably 85% or more, more preferably 90% or more, and even more preferably 95% or more with the sequence of SEQ ID NO: 12;
   a CDR2 including a sequence having a sequence identity of 80% or more, preferably 85% or more, more preferably 90% or more, and even more preferably 95% or more with the sequence of SEQ ID NO: 13; and
   a CDR3 including a sequence having a sequence identity of 80% or more, preferably 85% or more, more preferably 90% or more, and even more preferably 95% or more with the sequence of SEQ ID NO: 14.

In a certain embodiment, the first binding site that binds to LAG3 includes:
a heavy chain variable region including:
a CDR1 consisting of a sequence having a sequence identity of 80% or more, preferably 85% or more, more preferably 90% or more, and even more preferably 95% or more with the sequence of SEQ ID NO: 9;
a CDR2 consisting of a sequence having a sequence identity of 80% or more, preferably 85% or more, more preferably 90% or more, and even more preferably 95% or more with the sequence of SEQ ID NO: 10; and
a CDR3 consisting of a sequence having a sequence identity of 80% or more, preferably 85% or more, more preferably 90% or more, and even more preferably 95% or more with the sequence of SEQ ID NO: 11; and/or
a light chain variable region including:
   a CDR1 consisting of a sequence having a sequence identity of 80% or more, preferably 85% or more, more preferably 90% or more, and even more preferably 95% or more with the sequence of SEQ ID NO: 12;
   a CDR2 consisting of a sequence having a sequence identity of 80% or more, preferably 85% or more, more preferably 90% or more, and even more preferably 95% or more with the sequence of SEQ ID NO: 13; and
   a CDR3 consisting of a sequence having a sequence identity of 80% or more, preferably 85% or more, more preferably 90% or more, and even more preferably 95% or more with the sequence of SEQ ID NO: 14.

"Sequence identity" is determined by comparing two optimally aligned sequences over the entire region of the sequences to be compared. Here, in the two optimally aligned sequences to be compared, the sequences may have additions or deletions (e.g., gaps). The sequence identity can be calculated using a program such as FASTA, BLAST, or CLUSTAL W provided in public databases (e.g., DDBJ (http://www.ddbj.nig.ac.jp)). Alternatively, the sequence identity can be determined using commercially available sequence analysis software (for example, Vector NTI (registered trademark) software, GENETYX (registered trademark) ver. 12).

In a certain embodiment, the first binding site that binds to LAG3 includes:
a heavy chain variable region including:
a CDR1 including the sequence of SEQ ID NO: 9 in which 0, 1, or 2 amino acids are deleted, substituted, or added;
a CDR2 including the sequence of SEQ ID NO: 10 in which 0, 1, or 2 amino acids are deleted, substituted, or added; and
a CDR3 including the sequence of SEQ ID NO: 11 in which 0, 1, or 2 amino acids are deleted, substituted, or added; and/or
a light chain variable region including:
   a CDR1 including the sequence of SEQ ID NO: 12 in which 0, 1, or 2 amino acids are deleted, substituted, or added;
   a CDR2 including the sequence of SEQ ID NO: 13 in which 0, 1, or 2 amino acids are deleted, substituted, or added; and
   a CDR3 including the sequence of SEQ ID NO: 14 in which 0, 1, or 2 amino acids are deleted, substituted, or added.

In a certain embodiment, the first binding site that binds to LAG3 includes:
a heavy chain variable region including:
a CDR1 consisting of the sequence of SEQ ID NO: 9 in which 0, 1, or 2 amino acids are deleted, substituted, or added;
a CDR2 consisting of the sequence of SEQ ID NO: 10 in which 0, 1, or 2 amino acids are deleted, substituted, or added; and
a CDR3 consisting of the sequence of SEQ ID NO: 11 in which 0, 1, or 2 amino acids are deleted, substituted, or added; and/or
a light chain variable region including:
   a CDR1 consisting of the sequence of SEQ ID NO: 12 in which 0, 1, or 2 amino acids are deleted, substituted, or added;
   a CDR2 consisting of the sequence of SEQ ID NO: 13 in which 0, 1, or 2 amino acids are deleted, substituted, or added; and
   a CDR3 consisting of the sequence of SEQ ID NO: 14 in which 0, 1, or 2 amino acids are deleted, substituted, or added.

In a certain embodiment, the first binding site that binds to LAG3 includes: a heavy chain variable region that includes a sequence having a sequence identity of 80% or more, preferably 85% or more, more preferably 90% or more, and even more preferably 95% or more with the amino acid sequence of SEQ ID NO: 7; and/or a light chain variable region that includes a sequence having a sequence identity of 80% or more, preferably 85% or more, more preferably 90% or more, and even more preferably 95% or more with the amino acid sequence of SEQ ID NO: 8. In a further embodiment, the first binding site that binds to LAG3 includes: a heavy chain variable region that includes the amino acid sequence of SEQ ID NO: 7 in which 0 to 5 amino acids are deleted, substituted, or added; and/or a light chain variable region that includes the amino acid sequence of SEQ ID NO: 8 in which 0 to 5 amino acids are deleted, substituted, or added. These embodiments also encompass a first binding site that binds to LAG3 in which no modification has occurred to the CDRs of the heavy chain variable region and/or the light chain variable region. Specifically, these embodiments encompass a first binding site that binds to LAG3 that includes: a heavy chain variable region that includes a CDR1 consisting of the amino acid sequence of SEQ ID NO: 9, a CDR2 consisting of the amino acid sequence of SEQ ID NO: 10, and a CDR3 consisting of the amino acid sequence of SEQ ID NO: 11; and/or a light chain variable region that includes a CDR1 consisting of the amino acid sequence of SEQ ID NO: 12, a CDR2 consisting of the amino acid sequence of SEQ ID NO: 13, and a CDR3 consisting of the amino acid sequence of SEQ ID NO: 14.

In a certain embodiment, the first binding site that binds to LAG3 includes a heavy chain variable region including the CDRs 1 to 3 of the above-described light chain variable region and/or a light chain variable region including the CDRs 1 to 3 of the above-described heavy chain variable region.

In a certain embodiment, the first binding site that binds to LAG3 includes a heavy chain variable region including the amino acid sequence of SEQ ID NO: 7 and/or a light chain variable region including the amino acid sequence of SEQ ID NO: 8. In a further embodiment, the first binding site that binds to LAG3 includes a heavy chain variable region consisting of the amino acid sequence of SEQ ID NO: 7 and/or a light chain variable region consisting of the amino acid sequence of SEQ ID NO: 8.

In a certain embodiment, the first binding site that binds to LAG3 may be derived from a variable region of a known anti-LAG3 antibody, e.g., BMS-986016, LAG525, MK-4280, 11E3, 17B4, 3DS223H, REA351, REA776, 11C3C65, 7H2C65, C9B7W, or 631501.

In a certain embodiment, the first binding site that binds to LAG3 competes with any of the first binding sites specified above for the binding to LAG3. For example, the first binding site is derived from a variable region of an antibody that competes with any of the first binding sites specified above for the binding to LAG3. Competition can be confirmed, for example, by the competition assay described above.

In a certain embodiment, when a region of LAG3 to which any of the first binding sites specified above binds is specified, a region corresponding to the region in LAG3 of another type is specified, and a binding site that binds thereto, for example, a variable region of an antibody that binds thereto, can be used as the first binding site.

In a certain embodiment, the second binding site that binds to CD3 includes:
a heavy chain variable region including CDR1, CDR2, and CDR3 having the same amino acid sequences as those of CDR1, CDR2, and CDR3, respectively, included in a heavy chain variable region having the amino acid sequence of SEQ ID NO: 15; and/or
a light chain variable region including CDR1, CDR2, and CDR3 having the same amino acid sequences as those of CDR1, CDR2, and CDR3, respectively, included in a light chain variable region having the amino acid sequence of SEQ ID NO: 16.

In a certain embodiment, the second binding site that binds to CD3 includes:
a heavy chain variable region including a heavy chain CDR1 including the amino acid sequence of SEQ ID NO: 17, a heavy chain CDR2 including the amino acid sequence of SEQ ID NO: 18, and a heavy chain CDR3 including the amino acid sequence of SEQ ID NO: 19; and/or
a light chain variable region including a light chain CDR1 including the amino acid sequence of SEQ ID NO: 20, a light chain CDR2 including the amino acid sequence of SEQ ID NO: 21, and a light chain CDR3 including the amino acid sequence of SEQ ID NO: 22.

In a certain embodiment, the second binding site that binds to CD3 includes:
a heavy chain variable region that includes: a heavy chain CDR1 consisting of the amino acid sequence of SEQ ID NO: 17, a heavy chain CDR2 consisting of an amino acid sequence of SEQ ID NO: 18, and heavy chain CDR3 consisting of the amino acid sequence of SEQ ID NO: 19; and/or
a light chain variable region that includes a light chain CDR1 consisting of the amino acid sequence of SEQ ID NO: 20, a light chain CDR2 consisting of the amino acid sequence of SEQ ID NO: 21, and a light chain CDR3 consisting of the amino acid sequence of SEQ ID NO: 22.

In a certain embodiment, the second binding site that binds to CD3 includes:
a heavy chain variable region including:
a CDR1 including a sequence having a sequence identity of 80% or more, preferably 85% or more, more preferably 90% or more, and even more preferably 95% or more with the sequence of SEQ ID NO: 17;
a CDR2 including a sequence having a sequence identity of 80% or more, preferably 85% or more, more preferably 90% or more, and even more preferably 95% or more with the sequence of SEQ ID NO: 18; and
a CDR3 including a sequence having a sequence identity of 80% or more, preferably 85% or more, more preferably 90% or more, and even more preferably 95% or more with the sequence of SEQ ID NO: 19; and/or
a light chain variable region including:
   a CDR1 including a sequence having a sequence identity of 80% or more, preferably 85% or more, more preferably 90% or more, and even more preferably 95% or more with the sequence of SEQ ID NO: 20;
   a CDR2 including a sequence having a sequence identity of 80% or more, preferably 85% or more, more preferably 90% or more, and even more preferably 95% or more with the sequence of SEQ ID NO: 21; and
   a CDR3 including a sequence having a sequence identity of 80% or more, preferably 85% or more, more preferably 90% or more, and even more preferably 95% or more with the sequence of SEQ ID NO: 22.

In a certain embodiment, the second binding site that binds to CD3 includes:
a heavy chain variable region including:
a CDR1 consisting of a sequence having a sequence identity of 80% or more, preferably 85% or more, more preferably 90% or more, and even more preferably 95% or more with the sequence of SEQ ID NO: 17;
a CDR2 consisting of a sequence having a sequence identity of 80% or more, preferably 85% or more, more preferably 90% or more, and even more preferably 95% or more with the sequence of SEQ ID NO: 18; and
a CDR3 consisting of a sequence having a sequence identity of 80% or more, preferably 85% or more, more preferably 90% or more, and even more preferably 95% or more with the sequence of SEQ ID NO: 19; and/or
a light chain variable region including:
   a CDR1 consisting of a sequence having a sequence identity of 80% or more, preferably 85% or more, more preferably 90% or more, and even more preferably 95% or more with the sequence of SEQ ID NO: 20;
   a CDR2 consisting of a sequence having a sequence identity of 80% or more, preferably 85% or more, more preferably 90% or more, and even more preferably 95% or more with the sequence of SEQ ID NO: 21; and
   a CDR3 consisting of a sequence having a sequence identity of 80% or more, preferably 85% or more, more preferably 90% or more, and even more preferably 95% or more with the sequence of SEQ ID NO: 22.

In a certain embodiment, the second binding site that binds to CD3 includes:
a heavy chain variable region including:
a CDR1 including the sequence of SEQ ID NO: 17 in which 0, 1, or 2 amino acids are deleted, substituted, or added;
a CDR2 including the sequence of SEQ ID NO: 18 in which 0, 1, or 2 amino acids are deleted, substituted, or added; and
a CDR3 including the sequence of SEQ ID NO: 19 in which 0, 1, or 2 amino acids are deleted, substituted, or added; and/or
a light chain variable region including:
   a CDR1 including the sequence of SEQ ID NO: 20 in which 0, 1, or 2 amino acids are deleted, substituted, or added;
   a CDR2 including the sequence of SEQ ID NO: 21 in which 0, 1, or 2 amino acids are deleted, substituted, or added; and
   a CDR3 including the sequence of SEQ ID NO: 22 in which 0, 1, or 2 amino acids are deleted, substituted, or added.

In a certain embodiment, the second binding site that binds to CD3 includes:
a heavy chain variable region including:
a CDR1 consisting of the sequence of SEQ ID NO: 17 in which 0, 1, or 2 amino acids are deleted, substituted, or added;
a CDR2 consisting of the sequence of SEQ ID NO: 18 in which 0, 1, or 2 amino acids are deleted, substituted, or added; and
a CDR3 consisting of the sequence of SEQ ID NO: 19 in which 0, 1 or 2 amino acids are deleted, substituted, or added; and/or
a light chain variable region including:
   a CDR1 consisting of the sequence of SEQ ID NO: 20 in which 0, 1, or 2 amino acids are deleted, substituted, or added;
   a CDR2 consisting of the sequence of SEQ ID NO: 21 in which 0, 1, or 2 amino acids are deleted, substituted, or added; and
   a CDR3 consisting of the sequence of SEQ ID NO: 22 in which 0, 1, or 2 amino acids are deleted, substituted, or added.

In a certain embodiment, the second binding site that binds to CD3 includes: a heavy chain variable region that includes a sequence having a sequence identity of 80% or more, preferably 85% or more, more preferably 90% or more, and even more preferably 95% or more with the amino acid sequence of SEQ ID NO: 15; and/or a light chain variable region that includes a sequence having a sequence identity of 80% or more, preferably 85% or more, more preferably 90% or more, and even more preferably 95% or more with the amino acid sequence of SEQ ID NO: 16. In a further embodiment, the second binding site that binds to CD3 includes: a heavy chain variable region that includes the amino acid sequence of SEQ ID NO: 15 in which 0 to 5 amino acids are deleted, substituted, or added; and/or a light chain variable region that includes the amino acid sequence of SEQ ID NO: 16 in which 0 to 5 amino acids are deleted, substituted, or added. These embodiments also encompass a second binding site that binds to CD3 in which no modification has occurred to the CDRs of the heavy chain variable region and/or the light chain variable region. Specifically, these embodiments encompass a second binding site that binds to CD3 that includes: a heavy chain variable region that includes a CDR1 consisting of the amino acid sequence of SEQ ID NO: 17, a CDR2 consisting of the amino acid sequence of SEQ ID NO: 18, and a CDR3 consisting of the amino acid sequence of SEQ ID NO: 19; and/or a light chain variable region that includes a CDR1 consisting of the amino acid sequence of SEQ ID NO: 20, a CDR2 consisting of the amino acid sequence of SEQ ID NO: 21, and a CDR3 consisting of the amino acid sequence of SEQ ID NO: 22.

In a certain embodiment, the second binding site that binds to CD3 includes a heavy chain variable region including the CDRs 1 to 3 of the above-described light chain variable region and/or a light chain variable region including the CDRs 1 to 3 of the above-described heavy chain variable region.

In a certain embodiment, the second binding site that binds to CD3 includes a heavy chain variable region including the amino acid sequence of SEQ ID NO: 15 and/or a light chain variable region including the amino acid sequence of SEQ ID NO: 16. In a further embodiment, the second binding site that binds to CD3 includes a heavy chain variable region consisting of the amino acid sequence of SEQ ID NO: 15 and/or a light chain variable region consisting of the amino acid sequence of SEQ ID NO: 16.

In a certain embodiment, the second binding site that binds to CD3 can be derived from a variable region of a known anti-CD3 antibody, e.g., 2C11, 17A2, 500A2, KT3, OKT3 (ATCC accession number CRL8001) (U.S. Patent No. 4658019), 7D6, 12F6, 38.1, 89b1, 131F26, BL-A8, BW239/347, BW264/56, CD3-4B5, CLB-T3/3, CRIS-7, F111-409, G19-4.1, HIT3a, ICO-90, IP30, Leu-4, LY17.2G3, M-T301, M-T302, MEM-57, MEM-92, NU-T3, OKT3D, SMC2, T3, T3(2Ad2), T3/2Ad2A2, T3/2AD, T3(2ADA), T3/2T8-2F4, T3/RW2-4B6, T3/RW2-8C8, T10B9, T101-01, UCHT1, VIT3, VIT3b, X35-3, XXIII.46, XXIII.87, XXIII.141, YTH12.5, YTH12.5, CLB-T3.4.2, WT31, WT32, SPv-T3b, 11D8, M291, Leu4, 500A2, SP34, RIV-9, BH11, T2/30, AG3, BC3, OKT3γ1 (ala-ala) (US Patent No. 6491916), ChAglyCD3 (WO93/19196) or HUM291 (WO97/44362).

In a certain embodiment, the second binding site that binds to CD3 competes with any of the second binding sites specified above for the binding to CD3. In a certain embodiment, the second binding site that binds CD3ε competes with any of the second binding sites specified above for the binding to CD3ε. For example, the second binding site is derived from a variable region of an antibody that competes with any of the second binding sites specified above for the binding to CD3. Competition can be confirmed, for example, by the competition assay described above.

In a certain embodiment, when a region of CD3 to which any of the second binding sites specified above binds is specified, a region corresponding to the region in CD3 of another species is specified, and a binding site that binds to the region, for example, a variable region of an antibody that binds to the binding site, can be used as the second binding site.

In a certain embodiment, the second binding site that binds to CD8 includes:
a heavy chain variable region including CDR1, CDR2, and CDR3 having the same amino acid sequences as those of CDR1, CDR2, and CDR3, respectively, included in a heavy chain variable region having the amino acid sequence of SEQ ID NO: 23; and/or
a light chain variable region including CDR1, CDR2, and CDR3 having the same amino acid sequences as those of CDR1, CDR2, and CDR3, respectively, included in a light chain variable region having the amino acid sequence of SEQ ID NO: 24.

In a certain embodiment, the second binding site that binds to CD8 includes:
a heavy chain variable region including a heavy chain CDR1 including the amino acid sequence of SEQ ID NO: 25, a heavy chain CDR2 including the amino acid sequence of SEQ ID NO: 26, and a heavy chain CDR3 including the amino acid sequence of SEQ ID NO: 27; and/or
a light chain variable region including a light chain CDR1 including the amino acid sequence of SEQ ID NO: 28, a light chain CDR2 including the amino acid sequence of SEQ ID NO: 29, and a light chain CDR3 including the amino acid sequence of SEQ ID NO: 30.

In a certain embodiment, the second binding site that binds to CD8 includes:
a heavy chain variable region that includes: a heavy chain CDR1 consisting of the amino acid sequence of SEQ ID NO: 25, a heavy chain CDR2 consisting of an amino acid sequence of SEQ ID NO: 26, and heavy chain CDR3 consisting of the amino acid sequence of SEQ ID NO: 27; and/or
a light chain variable region that includes a light chain CDR1 consisting of the amino acid sequence of SEQ ID NO: 28, a light chain CDR2 consisting of the amino acid sequence of SEQ ID NO: 29, and a light chain CDR3 consisting of the amino acid sequence of SEQ ID NO: 30.

In a certain embodiment, the second binding site that binds to CD8 includes:
a heavy chain variable region including:
a CDR1 including a sequence having a sequence identity of 80% or more, preferably 85% or more, more preferably 90% or more, and even more preferably 95% or more with the sequence of SEQ ID NO: 25;
a CDR2 including a sequence having a sequence identity of 80% or more, preferably 85% or more, more preferably 90% or more, and even more preferably 95% or more with the sequence of SEQ ID NO: 26; and
a CDR3 including a sequence having a sequence identity of 80% or more, preferably 85% or more, more preferably 90% or more, and even more preferably 95% or more with the sequence of SEQ ID NO: 27; and/or
a light chain variable region including:
   a CDR1 including a sequence having a sequence identity of 80% or more, preferably 85% or more, more preferably 90% or more, and even more preferably 95% or more with the sequence of SEQ ID NO: 28;
   a CDR2 including a sequence having a sequence identity of 80% or more, preferably 85% or more, more preferably 90% or more, and even more preferably 95% or more with the sequence of SEQ ID NO: 29; and
   a CDR3 including a sequence having a sequence identity of 80% or more, preferably 85% or more, more preferably 90% or more, and even more preferably 95% or more with the sequence of SEQ ID NO: 30.

In a certain embodiment, the second binding site that binds to CD8 includes:
a heavy chain variable region including:
a CDR1 consisting of a sequence having a sequence identity of 80% or more, preferably 85% or more, more preferably 90% or more, and even more preferably 95% or more with the sequence of SEQ ID NO: 25;
a CDR2 consisting of a sequence having a sequence identity of 80% or more, preferably 85% or more, more preferably 90% or more, and even more preferably 95% or more with the sequence of SEQ ID NO: 26; and
a CDR3 consisting of a sequence having a sequence identity of 80% or more, preferably 85% or more, more preferably 90% or more, and even more preferably 95% or more with the sequence of SEQ ID NO: 27; and/or
a light chain variable region including:
   a CDR1 consisting of a sequence having a sequence identity of 80% or more, preferably 85% or more, more preferably 90% or more, and even more preferably 95% or more with the sequence of SEQ ID NO: 28;
   a CDR2 consisting of a sequence having a sequence identity of 80% or more, preferably 85% or more, more preferably 90% or more, and even more preferably 95% or more with the sequence of SEQ ID NO: 29; and
   a CDR3 consisting of a sequence having a sequence identity of 80% or more, preferably 85% or more, more preferably 90% or more, and even more preferably 95% or more with the sequence of SEQ ID NO: 30.

In a certain embodiment, the second binding site that binds to CD8 includes:
a heavy chain variable region including:
a CDR1 including the sequence of SEQ ID NO: 25 in which 0, 1, or 2 amino acids are deleted, substituted, or added;
a CDR2 including the sequence of SEQ ID NO: 26 in which 0, 1, or 2 amino acids are deleted, substituted, or added; and
a CDR3 including the sequence of SEQ ID NO: 27 in which 0, 1, or 2 amino acids are deleted, substituted, or added; and/or
a light chain variable region including:
   a CDR1 including the sequence of SEQ ID NO: 28 in which 0, 1, or 2 amino acids are deleted, substituted, or added;
   a CDR2 including the sequence of SEQ ID NO: 29 in which 0, 1, or 2 amino acids are deleted, substituted, or added; and
   a CDR3 including the sequence of SEQ ID NO: 30 in which 0, 1, or 2 amino acids are deleted, substituted, or added.

In a certain embodiment, the second binding site that binds to CD8 includes:
a heavy chain variable region including:
a CDR1 consisting of the sequence of SEQ ID NO: 25 in which 0, 1, or 2 amino acids are deleted, substituted, or added;
a CDR2 consisting of the sequence of SEQ ID NO: 26 in which 0, 1, or 2 amino acids are deleted, substituted, or added; and
a CDR3 consisting of the sequence of SEQ ID NO: 27 in which 0, 1, or 2 amino acids are deleted, substituted, or added; and/or
a light chain variable region including:
   a CDR1 consisting of the sequence of SEQ ID NO: 28 in which 0, 1, or 2 amino acids are deleted, substituted, or added;
   a CDR2 consisting of the sequence of SEQ ID NO: 29 in which 0, 1, or 2 amino acids are deleted, substituted, or added; and
   a CDR3 consisting of the sequence of SEQ ID NO: 30 in which 0, 1, or 2 amino acids are deleted, substituted, or added.

In a certain embodiment, the second binding site that binds to CD8 includes: a heavy chain variable region that includes a sequence having a sequence identity of 80% or more, preferably 85% or more, more preferably 90% or more, and even more preferably 95% or more with the amino acid sequence of SEQ ID NO: 23; and/or a light chain variable region that includes a sequence having a sequence identity of 80% or more, preferably 85% or more, more preferably 90% or more, and even more preferably 95% or more with the amino acid sequence of SEQ ID NO: 24. In a further embodiment, the second binding site that binds to CD8 includes: a heavy chain variable region that includes the amino acid sequence of SEQ ID NO: 23 in which 0 to 5 amino acids are deleted, substituted, or added; and/or a light chain variable region that includes the amino acid sequence of SEQ ID NO: 24 in which 0 to 5 amino acids are deleted, substituted, or added. These embodiments also encompass a second binding site that binds to CD8 in which no modification has occurred to the CDRs of the heavy chain variable region and/or the light chain variable region. Specifically, these embodiments encompass a second binding site that binds to CD8 that includes: a heavy chain variable region that includes a CDR1 consisting of the amino acid sequence of SEQ ID NO: 25, a CDR2 consisting of the amino acid sequence of SEQ ID NO: 26, and a CDR3 consisting of the amino acid sequence of SEQ ID NO: 27; and/or a light chain variable region that includes a CDR1 consisting of the amino acid sequence of SEQ ID NO: 28, a CDR2 consisting of the amino acid sequence of SEQ ID NO: 29, and a CDR3 consisting of the amino acid sequence of SEQ ID NO: 30.

In a certain embodiment, the second binding site that binds to CD8 includes a heavy chain variable region including the CDR1 to 3 of the above-described light chain variable region and/or a light chain variable region including the CDR1 to 3 of the above-described heavy chain variable region.

In a certain embodiment, the second binding site that binds to CD8 includes a heavy chain variable region including the amino acid sequence of SEQ ID NO: 23 and/or a light chain variable region including the amino acid sequence of SEQ ID NO: 24. In a further embodiment, the second binding site that binds to CD8 includes a heavy chain variable region consisting of the amino acid sequence of SEQ ID NO: 23 and/or a light chain variable region consisting of the amino acid sequence of SEQ ID NO: 24.

In a certain embodiment, the second binding site that binds to CD8 may be derived from a variable region of a known anti-CD8 antibody, e.g., YTS169, cM-T807, T8/Leu2/SK1, RPA-T8, HIT8a, OKT8 (Japanese Translation of PCT International Application Publication No. 2011-522835), 53-6.7, 53-5.8, 5H10, YTS-156, KT15, LT8, or CA-8.

In a certain embodiment, the second binding site that binds to CD8 competes with any of the second binding sites specified above for the binding to CD8. In a certain embodiment, the second binding site that binds to CD8α competes with any of the second binding sites specified above for the binding to CD8α. For example, the second binding site is derived from a variable region of an antibody that competes with any of the second binding sites specified above for the binding to CD8. Competition can be confirmed, for example, by the competition assay described above.

In a certain embodiment, when a region of CD8 to which any of the second binding sites specified above binds is specified, a region corresponding to the region in CD8 of another species is specified, and a binding site that binds to the region, for example, a variable region of an antibody that binds to the binding site, can be used as the second binding site.

A bispecific molecule may have a structure that conforms to the format of the multispecific molecule known in the art. Examples of the multispecific molecule are disclosed in, for example, The coming of Age of Engineered Multivalent Antibodies, Nunez-Prado et al., Drug Discovery Today Vol 20 Number 5 Mar 2015, page 588-594, D. Holmes, Nature Rev Drug Disc Nov 2011: 10, 798, Chan and Carter, Nature Reviews Immunology vol 10, May 2010, 301, and Japanese Translation of PCT International Application Publication No. 2017-522023, incorporated herein by reference.

In a certain embodiment, the format of the bispecific molecule is selected from diabody, bispecific sc(Fv)₂, bispecific minibody, bispecific F(ab')₂, bispecific antibody, covalent diabody (bispecific DART) (WO2006/113665 or WO2008/157379), bispecific (FvCys)₂ (J. Immunol., 1992, Vol. 149, No. 1, p. 120-126), bispecific F(ab'-zipper)₂ (J. Immunol., 1992, Vol. 148, No. 5, p. 1547-1553), bispecific (Fv-zipper)₂ (Biochemistry, 1992, Vol. 31, No. 6, p. 1579-1584), bispecific triple-chain antibody (Proc. Natl. Acad. Sci. USA, 1993, Vol. 90, No. 14, p. 6444-6448), and bispecific mAb² (www.f-star.com/technology_mab.html). In a certain embodiment, the format of the bispecific molecule is selected from diabody, tandem scFv, scDiabody, FabFv, Fab'Fv, FabdsFv, Fab-scFv, Fab-dsscFv, Fab-(dsscFv)2, diFab, diFab', scFv-Fc, tandem scFv-Fc, scDiabody-Fc, scDiabody-CH3, Ig-scFv, and scFv-Ig (Japanese Translation of PCT International Application Publication No. 2017-526616). In a certain embodiment, the bispecific molecule is selected from diabody, tandem scFv, and scDiabody. In a certain embodiment, the bispecific molecule is an scDiabody. In a certain embodiment, the bispecific molecule is a bispecific antibody, and preferably a bispecific monoclonal antibody. In a certain embodiment, a bispecific molecule described herein includes a constant region. The bispecific antibody can be an antibody of any immunoglobulin class. Examples of the immunoglobulin class include IgA, IgD, IgE, IgG, and IgM, and examples of the subclass (isotype) of the immunoglobulin class include IgG1, IgG2, IgG3, IgG4, IgA1, and IgA2. In a certain embodiment, the bispecific antibody is of the IgG subclass, e.g., the IgG1 or IgG4 subclass.

A diabody is a dimer composed of two polypeptide chains, and in each polypeptide chain, a heavy chain variable region (V_{H}) and a light chain variable region (V_{L}) are linked by a linker in such a manner that they cannot associate with each other in the same chain (Proc. Natl. Acad. Sci. USA, 1993, Vol. 90, No. 14, p. 6444-6448). A diabody has two antigen-binding sites, as V_{H} and V_{L} on one polypeptide chain are associated with V_{L} and V_{H} on the other polypeptide chain, respectively. The linker is not particularly limited as long as it does not inhibit the expression of V_{H} and V_{L} and the formation of a diabody, and may be, for example, Ser, (Gly)n-Ser, Ser-(Gly)n, ((Gly)4-Ser)n, (Ser-(Gly)₄)ₙ [n represents an integer of 1 to 6], or the like (J. Immunol. Meth., 1999, Vol. 231, p. 177-189). Typically, the peptide linker is short enough that the V_{H} and V_{L} in the polypeptide chain cannot associate with each other. For example, it is about 2 to 12, about 3 to 10, or in particular, about 5 amino acid residues in length, or alternatively, it has a structure in which the V_{H} and V_{L} in the polypeptide chain cannot associate.

A bispecific sc(Fv)₂ is a polypeptide chain in which the V_{H}s and V_{L}s of two antibodies recognizing different antigens are linked via linkers in a single chain (J. Biological Chemistry, 1994, 269: 199-206). Here, when V_{H} and V_{L} of an antibody recognizing an antigen a, and V_{H} and V_{L} of another antibody recognizing an antigen b different from the antigen a, are denoted as V_{H}a, V_{L}a, V_{H}b, and V_{L}b, respectively, and peptide linkers are denoted as (L₁), (L₂), and (L₃), then, examples of the form of the bispecific sc(Fv)₂ include:
(1) V_{H}a-(L₁)-V_{L}a-(L₂)-V_{H}b-(L₃)-V_{L}b;
(2) V_{H}a-(L₁)-V_{L}a-(L₂)-V_{L}b-(L₃)-V_{H}b;
(3) V_{L}a-(L₁)-V_{H}a-(L₂)-V_{H}b-(L₃)-V_{L}b;
(4) V_{L}a-(L₁)-V_{H}a-(L₂)-V_{L}b-(L₃)-V_{H}b;
(5) V_{H}a-(L₁)-V_{H}b-(L₂)-V_{L}b-(L₃)-V_{L}a;
(6) V_{H}a-(L₁)-V_{L}b-(L₂)-V_{H}b-(L₃)-V_{L}a;
(7) V_{L}a-(L₁)-V_{L}b-(L₂)-V_{H}b-(L₃)-V_{H}a; and
(8) V_{L}a-(L₁)-V_{H}b-(L₂)-V_{L}b-(L₃)-V_{H}a,
in each of which V_{H} and V_{L} as well as the linkers are arranged in the stated order from the N-terminal side. The bispecific sc(Fv)₂ is formed by association of V_{H}a and V_{L}a and association of V_{H}b and V_{L}b. Here, the peptide linker is not particularly limited as long as it does not inhibit the expression and formation of the bispecific sc(Fv)₂, and may be, for example, Ser, (Gly)ₙ-Ser, Ser-(Gly)ₙ, ((Gly)₄-Ser)ₙ, (Ser-(Gly)₄)ₙ [n represents an integer of 1 to 6], Ser-Ser-Ala-Asp-Asp-Ala-Lys-Lys-Asp-Ala-Ala-Lys-Lys-(Asp-Asp-Ala-Lys-Lys)₂-Asp-Ala, or the like.

The bispecific sc(Fv)₂ in the forms (1) to (4) above is particularly referred to as tandem scFv. Typically, the peptide linker (L₂) of a tandem scFv is short enough that two variable regions adjacent thereto cannot associate with each other. For example, it is about 2 to 12, about 3 to 10, or in particular, about 5 amino acid residues in length, or alternatively, it has a structure in which two variable regions adjacent thereto cannot associate with each other. The peptide linkers (L₁) and (L₃) of the tandem scFv have a length and structure by which two variable regions adjacent to each peptide linker can associate with each other. For example, they are about 10 to 25, about 13 to 20, or in particular, about 15 amino acid residues in length. (L₁) and (L₃) may be the same or different.

The bispecific sc(Fv)₂ in the forms (5) to (8) above is particularly referred to as an scDiabody. Typically, the peptide linkers (L₁) and (L₃) of an scDiabody are short enough that two variable regions adjacent thereto cannot associate with each other. For example, it is about 2 to 12, about 3 to 10, or in particular, about 5 amino acid residues in length, or alternatively, it has a structure in which two variable regions adjacent thereto cannot associate with each other. (L₁) and (L₃) may be the same or different. The peptide linker (L₂) of the tandem scFv has a length and structure by which two variable regions adjacent thereto can associate with each other. For example, it is about 10 to 25 or about 13 to 20, in particular, about 15 amino acid residues in length.

When the bispecific molecule of the present disclosure is a tandem scFv or scDiabody, the first binding site binding to LAG3 may be composed of V_{H}a and V_{L}a of the above formula and the second binding site binding to CD3 or CD8 may be composed of V_{H}b and V_{L}b. Alternatively, the first binding site binding to LAG3 may be composed of V_{H}b and V_{L}b of the above formula, and the second binding site binding to CD3 or CD8 may be composed of V_{H}a and V_{L}a.

The bispecific antibody is an intact antibody in which a heavy chain/light chain complex of an antibody recognizing two different antigens is obtained by covalent bond such as a disulfide bond or the like. A bispecific antibody can be produced, for example, from hybridomas produced by the hybrid hybridoma method (U.S. Patent No. 4474893). In addition, it can be produced by causing four kinds of cDNAs encoding heavy chains and light chains of antibodies that recognize different antigens, respectively, to be expressed in mammalian cells and to secrete proteins. In a certain embodiment, the bispecific antibody is of the IgG subclass, e.g., the IgG1 or IgG4 subclass.

The bispecific F(ab')₂ is a low molecular weight antibody in which Fab' fragments of antibodies recognizing two different antigens, respectively, are covalently bonded by a disulfide bond or the like. Here, the Fab' fragment is an antibody fragment prepared by cleaving a disulfide bond between two heavy chains of F(ab')₂ obtained by digesting an intact antibody with pepsin. Bispecific F(ab')₂ can be produced, for example, by maleimidizing a Fab' fragment prepared from one antibody with o-phenylenedimaleimide, and reacting the maleimidized Fab' fragment prepared from the other antibody (Cancer Research 1997, 57: 4008-4014). In addition, a method of chemically bonding a Fab' fragment-thionitrobenzoate derivative and one antibody fragment such as Fab'-SH is also known (Science 1985, 229: 81-83).

A bispecific minibody is a low molecular weight antibody in which small molecule antibody fragments modified so that constant region CH3 domains of antibodies are linked to scFvs that recognize different antigens, respectively, are covalently bonded by disulfide bonds or the like on the CH3 domains (Biochemistry, 1992, Vo. 31, No. 6, p. 1579-1584). Here, the scFv is a single-chain modified low molecular weight antibody fragment having a form in which V_{H} and V_{L} are linked by a peptide linker or the like (J. Immunol. Meth., 1999, Vol. 231, p. 177-189).

Bispecific molecules in these formats can be produced using genes encoding portions corresponding to V_{H} and V_{L}, respectively, constituting the antigen binding sites. The genes encoding the V_{H} and V_{L} portions can be obtained mainly from an antibody gene library, or by gene cloning from hybridomas producing monoclonal antibodies.

The bispecific molecule can be produced by inserting an isolated cDNA encoding the bispecific molecule into an expression vector and causing the same to be expressed and secreted in a host cell. For example, in the case of a diabody, the vector expressing the single-chain peptides constituting the diabody can be produced by linking cDNAs encoding portions corresponding to V_{H} and V_{L} recognizing different antigens in frame so that a DNA encoding a peptide linker is interposed therebetween, and inserting the same into an expression vector. The DNAs expressing the single-stranded peptides, respectively, may be inserted into the same expression vector, or may be inserted into separate expression vectors. When this expression vector is introduced into an appropriate host cell and expressed, the diabody can be directly secreted from the host cell. In addition, in the case of a bispecific sc(FV)₂, it can be produced by, for example, linking cDNAs encoding V_{H} and V_{L} recognizing one antigen, cDNAs encoding V_{H} and V_{L} recognizing the other antigen, and cDNAs encoding peptide linkers in frame, and inserting the same into an expression vector. When this expression vector is introduced into an appropriate host cell and expressed, the bispecific sc(Fv)₂ can be directly secreted from the host cell. Here, examples of the expression vector that can be used for expression of a diabody or a bispecific sc(Fv)₂ include pEBMulti-Neo (Wako), pCANTAB5E (manufactured by GE Healthcare Biosciences) and the like.

As the host cell, for example, eukaryotic cells such as animal cells, plant cells, and fungal cells can be used. Examples of the animal cells include mammalian cells (e.g., CHO, COS, NIH3T3, myeloma, BHK (baby hamster kidney), HeLa, Vero, 293T, platE), amphibian cells (e.g., Xenopus oocytes), or insect cells (e.g., Sf9, Sf21, Tn5). Examples of fungal cells include yeast (e.g., the genus Saccharomyces, such as Saccharomyces cerevisiae), filamentous fungi (e.g., the genus Aspergillus, such as Aspergillus niger), and the like. In addition, prokaryotic cells such as colon bacillus (Escherichia coli (E. coli)) (e.g., JM109, DH5α, HB101, etc.) and Bacillus subtilis can also be used as host cells. The vector can be introduced into the host cell by, for example, the calcium phosphate method, the DEAE dextran method, the electroporation method, a lipofection and the like.

The present disclosure also provides a polynucleotide encoding a bispecific molecule, an expression vector including the polynucleotide, and a transformed cell including the polynucleotide or the expression vector.

The bispecific molecule may be bound to a polymer in order to, for example, extend the half-life or improve stability, the polymer being polyethylene glycol (PEG), polypropylene glycol, polyoxyalkylene, a copolymer of polyethylene glycol and polypropylene glycol, or the like. In addition, an additional sequence such as a signal sequence may be included.

The amino acid residues of the bispecific molecule may be modified by a known method. For example, the functional group in the side chain of the amino acid residue, the amino group of the N-terminal amino acid, or the carboxyl group of the C-terminal amino acid may be subjected to esterification, alkylation, halogenation, phosphorylation, or the like. In addition, various substances can be bound to the N-terminus and/or C-terminus of the bispecific molecule. For example, an amino acid, a peptide, an analog thereof, or the like may be bound. For example, a tag such as a histidine tag or a FLAG tag may be added. When these substances are bound to the bispecific molecule, these substances may be processed by, for example, an in vivo enzyme or the like, or by a process such as intracellular processing, and finally generate the bispecific molecule. These substances may modulate the solubility of the bispecific molecule, improve its stability such as a protease resistance action, or deliver the bispecific molecule specifically to a predetermined tissue or organ, for example.

Since the bispecific molecules or immunosuppressants disclosed herein have low toxicity, they can be safely used as pharmaceutical products.

### [Application to pharmaceutical products]

As demonstrated by the examples, the bispecific molecules disclosed herein can suppress immunity. Thus, bispecific molecules may be used as immunosuppressants. The bispecific molecules disclosed herein can be used for the prevention and/or treatment of diseases characterized by enhanced immunity.

Examples of the disease characterized by enhanced immunity include autoimmune diseases, allergic diseases and graft-versus-host diseases. Examples of the autoimmune disease include Behcet's disease, systemic lupus erythematosus, multiple sclerosis (systemic sclerosis, progressive systemic sclerosis), scleroderma, polymyositis, dermatomyositis, periarteritis nodosa (polyarteritis nodosa, microscopic polyangiitis), aortitis syndrome (Takayasu's arteritis), malignant rheumatoid arthritis, rheumatoid arthritis, juvenile idiopathic arthritis, Wegener's granulomatosis, mixed connective tissue disease, Sjogren's syndrome, Adult-onset Still's disease, allergic granulomatous angiitis, hypersensitivity vasculitis, Cogan's syndrome, RS3PE syndrome, temporal arteritis, polymyalgia rheumatica, fibromyalgia, antiphospholipid antibody syndrome, eosinophilic fasciitis, IgG4-related diseases (e.g., primary sclerosing cholangitis, autoimmune pancreatitis), Guillain-Barre syndrome, myasthenia gravis, chronic atrophic gastritis, autoimmune hepatitis, primary biliary cirrhosis, aortitis syndrome, Goodpasture syndrome, rapidly progressive glomerulonephritis, megaloblastic anemia, autoimmune hemolytic anemia, autoimmune neutropenia, idiopathic thrombocytopenic purpura, Basedow's disease (hyperthyroidism), Hashimoto disease, autoimmune adrenal insufficiency, primary hypothyroidism, idiopathic Addison disease (chronic hypoadreno corticism), type I diabetes, slowly progressive type I diabetes (latent autoimmune diabetes in adults), chronic discoid lupus erythematosus, circumscribed scleroderma, psoriasis, psoriatic arthritis, pemphigus, pemphigoid, gestational herpes, linear IgA bullous dermatosis, acquired epidermolysis bullosa, alopecia areata, white spots, vitiligo vulgaris, atopic dermatitis, neuromyelitis optica, Chronic inflammatory demyelinating polyneuropathy, sarcoidosis, bullous pemphigoid, giant cell arteritis, amyotrophic lateral sclerosis, eosinophilic granulomatosis with polyangiitis, Harada disease, autoimmune optic neuropathy, idiopathic azoospermia, habitual abortion, inflammatory bowel disease (e.g., ulcerative colitis, Crohn's disease), and celiac disease. In a certain embodiment, the autoimmune disease is type I diabetes, multiple sclerosis, systemic lupus erythematosus, or rheumatoid arthritis. In a certain embodiment, the autoimmune disease is multiple sclerosis. Examples of the allergic disease include asthma, atopic dermatitis, rhinitis, conjunctivitis, and hay fever.

As used herein, "treating" or "treatment" means reducing or eliminating the cause of a disease in a subject with the disease, delaying or stopping the progression of the disease, reducing, alleviating, improving, and/or eliminating its symptoms, or suppressing the exacerbation of its symptoms.

As used herein, "preventing" or "prevention" means preventing the development of a disease, or reducing the likelihood of developing the disease in a subject, especially in a subject who is highly likely to develop the disease but has not yet developed the disease. It also encompasses the prevention of recurrence. Subjects who are likely to develop autoimmune diseases or allergic diseases but have not yet developed the diseases encompass subjects with enhanced immunity; subjects with genetic predispositions to autoimmune diseases or allergic diseases; and subjects who have been affected and cured of autoimmune or allergic diseases in the past. Subjects who may develop graft-versus-host diseases but have not yet developed encompass those who undergo an organ transplant.

Immunosuppressants, or prophylactic and/or therapeutic agents for diseases characterized by enhanced immunity, can be administered to animals, typically mammals (e.g., humans, mice, rats, hamsters, rabbits, cats, dogs, cows, sheep, and monkeys, etc.), among which humans are particularly preferred. Also preferred are subjects that require immunosuppression, or the prevention and/or treatment, and are particularly those who require the treatment (for example, patients).

The dose of the active ingredient is appropriately selected depending on the administration method, the age, body weight, health condition and the like of the administration target. For example, 0.1 µg/kg to 300 mg/kg per day can be administered to an adult, continuously over a period ranging from 30 minutes to 24 hours a day, once to several times a day, or once to several times one or several days, or one or several weeks for example, once every 1 to 3 weeks, though not limited to this. The administration method also is appropriately selected depending on the age, body weight, health condition and the like of the administration target. The administration method may be oral administration or parenteral administration, but parenteral administration is preferable. Examples of parenteral administration include subcutaneous administration, intradermal administration, intraperitoneal administration, intramuscular administration, and intravenous administration, but intravenous administration is preferable.

Immunosuppressants, or prophylactic and/or therapeutic agents for diseases characterized by enhanced immunity, can be formulated by conventional methods. The formulation may contain a variety of pharmaceutically acceptable substances for formulation, as required in formulation. The substance for formulation can be appropriately selected depending on the dosage form of the formulation, and examples of the same include a buffering agent, a surfactant, a stabilizer, a preservative, an excipient, a diluent, an additive, a disintegrant, a binder, a coating agent, a lubricant, a lubricating agent, and a solubilizer. For example, immunosuppressants can be formulated as injections or infusions. The injection or the infusion can be in the sterilized aqueous solution form, the suspension form, or the emulsion form, or in the solid dosage form or lyophilized form for use in a state of being dissolved, suspended, or emulsified in a sterilized liquid. The sterilized liquid can be, for example, water for injection, saline, glucose solution, or isotonic solution. Immunosuppressants can also be formulated in such a manner that sustained release or controlled release of the active ingredient is achieved. Methods for producing these formulations are well known in the art.

The formulation may contain a pharmaceutically acceptable carrier. In the present disclosure, a "pharmaceutically acceptable carrier" contains a certain arbitrary substance that is non-reactive with the immune system of interest, which, when combined with an active ingredient, can retain the biological activity of that ingredient. Examples of the pharmaceutically acceptable carrier include stabilizers, solubilizers, suspending agents, emulsifiers, soothing agents, buffers, preservatives, pH adjusters and antioxidants. As the stabilizer, for example, the following can be used: various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, and dibutylhydroxytoluene. As the solubilizer, for example, the following can be used: alcohols (e.g., ethanol), polyalcohols (e.g., propylene glycol, polyethylene glycol), and nonionic surfactants (e.g., polysorbate 20(registered trademark), polysorbate 80(registered trademark), HCO-50, etc.). As the suspending agent, for example, the following can be used: glycerin monostearate, aluminum monostearate, methyl cellulose, carboxymethyl cellulose, hydroxymethyl cellulose, and sodium lauryl sulfate. As the emulsifier, for example, the following can be used: gum arabic, sodium alginate, and tragacanth. As the soothing agent, for example, the following can be used: benzyl alcohol, chlorobutanol, and sorbitol can be used. As the buffer, for example, the following can be used: a phosphate buffer solution, an acetate buffer solution, a borate buffer solution, a carbonate buffer solution, a citrate buffer solution, a Tris buffer solution, a glutamic acid buffer solution, and an epsilon aminocaproic acid buffer solution. As the preservative, for example, the following can be used: methyl paraoxybenzoate, ethyl paraoxybenzoate, propyl paraoxybenzoate, butyl paraoxybenzoate, chlorobutanol, benzyl alcohol, benzalkonium chloride, sodium dehydroacetate, sodium edetate, boric acid, and borax. As the preservative, for example, benzalkonium chloride, paraoxybenzoic acid, and chlorobutanol can be used. As the pH adjuster, for example, hydrochloric acid, sodium hydroxide, phosphoric acid, and acetic acid can be used. As antioxidants, for example, the following can be used: (1) water-soluble antioxidants such as ascorbic acid, cysteine hydrochloride, sodium bisulfate, sodium metabisulfite, sodium sulfite, etc.; (2) oil-soluble antioxidants such as ascorbyl palmitate, butylated hydroxyanisole, butylated hydroxytoluene, lecithin, propyl gallate, α-tocopherol, etc.; and (3) metal chelating agents such as citric acid, ethylenediaminetetraacetic acid, sorbitol, tartaric acid, phosphoric acid.

An infusion solution for injection or infusion can be produced through a process of: sterilizing the same in the final step, or applying an aseptic technique such as filtering with a filter or the like and sterilizing the same; and then, filling the same in a sterile container. A vacuum-dried and lyophilized sterile powder (which may contain a pharmaceutically acceptable carrier powder) may be dissolved in a suitable solvent before use, so as to be used as the infusion solution for injection or infusion.

Immunosuppressants, or prophylactic and/or therapeutic agents for preventing and/or treating diseases characterized by enhanced immunity, can be used alone, or can be used in combination with one or more additional active ingredients, in particular with an active ingredient for immunosuppression. "Combination" of ingredients is not limited to the use of a dosage form containing all ingredients, and the use of a combination of dosage forms containing the ingredients respectively. It also encompasses administering all of the component simultaneously or administering the same with a delay as for a certain component, as long as they are used for immunosuppression, or the treatment and/or prevention of diseases characterized by enhanced immunity. In a case where a certain component is administered with a delay, there may be a period during which the components are co-administered. It is also possible to combine two or more additional active ingredients. The combined use enables, for example, to complement the prophylactic and/or therapeutic effects of other active ingredients, and to maintain and/or reduce the dose or the frequency of administration. Examples of the active ingredients suitable for the combined use include anti-inflammatory agents, antibacterial agents, antifungal agents, antiviral agents, immunosuppressants, and molecular targeting agents.

For example, when the immunosuppressant of the present disclosure, or the prophylactic and/or therapeutic agent of the present disclosure for a disease characterized by enhanced immunity, is applied to the prevention and/or treatment of type I diabetes, it may be used in combination with any one or more agents selected from the following: insulin preparations (e.g., human insulin, insulin glargine, insulin lispro, insulin detemir, insulin aspart); sulfonylureas (e.g., glibenclamide, gliclazide, glimepiride); fast-acting insulin secretagogue (e.g., nateglinide); biguanide preparations (e.g. metformin),
insulin sensitizers (e.g., pioglitazone); α-glucosidase inhibitors (e.g., acarbose, voglibose); therapeutic agents for diabetic neuropathy (e.g., epalrestat, mexiletine, imidapril); GLP-1 analogs (e.g., liraglutide, exenatide, lixisenatide); and DPP-4 inhibitors (e.g. sitagliptin, vildagliptin, alogliptin).

In addition, for example, when the immunosuppressant of the present disclosure, or the prophylactic and/or therapeutic agent of the present disclosure for a disease characterized by enhanced immunity, is applied to the prevention and/or treatment of multiple sclerosis, it may be used in combination with any one or more agents selected from the following: steroid drugs (e.g., cortisone acetate, hydrocortisone, hydrocortisone sodium phosphate, hydrocortisone sodium succinate, fludrocortisone acetate, prednisolone, prednisolone acetate, prednisolone sodium succinate, butyl prednisolone, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone phosphate, dexamethasone palmitate, paramethasone acetate, betamethasone); interferon β-1a, interferon β-1b, glatiramer acetate, mitoxantrone, azathiopurine, cyclophosphamide, cyclosporine, methotrexate, cladribine, adrenocorticotrophic hormone (ACTH), corticotropin, mizoribine, tacrolimus, fingolimod and alemtuzumab, etc.

In addition, for example, when the immunosuppressant or the prophylactic and/or therapeutic agent of the present disclosure for diseases characterized by enhanced immunity is applied to the prevention and/or treatment of systemic lupus erythematosus, it may be used in combination with one or more selected from the following: a steroid drug (for example, the steroid drug described above), immunosuppressants (e.g., cyclosporine, tacrolimus, fingolimod, etc.) and belimumab.

In addition, for example, when the immunosuppressant of the present disclosure, or the prophylactic and/or therapeutic agent of the present disclosure for a disease characterized by enhanced immunity, is applied to the prevention and/treatment of rheumatoid arthritis, it may be used in combination with one or more selected from the following: steroid drugs (for example, the steroid drugs described above); anti-rheumatic drugs (e.g., methotrexate, sulfasalazine, bucillamine, leflunomide, mizoribine, tacrolimus, etc.) or anti-cytokine drugs (e.g., infliximab, adalimumab, tocilizumab, etanercept, golimumab, certolizumab, etc.); and abatacept.

When applied to the prevention and/or treatment of other autoimmune diseases, allergic diseases or graft-versus-host diseases, the immunosuppressant of the present disclosure, or the prophylactic and/or therapeutic agent of the present disclosure for a disease characterized by enhanced immunity, may be used in combination with any one or more of the other agents described above.

In a certain aspect, provided is a method of immunosuppression including administering, to a subject in need of immunosuppression, an effective amount of a bispecific molecule including a first binding site that specifically binds to LAG3 and a second binding site that specifically binds to CD3 or CD8. As used herein, the term "effective amount" means an amount with which an effect of suppressing immunity in a subject can be exerted.

In a certain aspect, provided is a bispecific molecule for use in immunosuppression, including a first binding site that specifically binds to LAG3 and a second binding site that specifically binds to CD3 or CD8.

In a certain aspect, provided is use of a bispecific molecule in the production of a pharmaceutical composition for immunosuppression, the bispecific molecule including a first binding site that specifically binds to LAG3 and a second binding site that specifically binds to CD3 or CD8.

In a certain aspect, provided is a method for preventing and/or treating a disease characterized by enhanced immunity, the method including administering an effective amount of a bispecific molecule including a first binding site that specifically binds to LAG3 and a second binding site that specifically binds to CD3 or CD8, to a subject in need thereof.

In a certain aspect, provided is a bispecific molecule for use in the prevention and/or treatment of a disease characterized by enhanced immunity, the bispecific molecule including a first binding site that specifically binds to LAG3 and a second binding site that specifically binds to CD3 or CD8.

In a certain aspect, provided is a bispecific molecule for use in the production of a prophylactic and/or therapeutic agent for a disease characterized by enhanced immunity, the bispecific molecule including a first binding site that specifically binds to LAG3 and a second binding site that specifically binds to CD3 or CD8.

In yet another aspect, an anti-LAG3 antibody or a fragment thereof is provided. Anti-LAG3 antibodies can suppress the function of LAG3, thereby activating immunity. The anti-LAG3 antibody may be an antibody described above with respect to the obtainment of the binding site of the bispecific molecule, for example, a monoclonal antibody.

In a certain embodiment, the anti-LAG3 antibody binds to a region including an amino acid corresponding to asparagine at position 54, phenylalanine at position 55, valine at position 61, and/or isoleucine at position 62 of mouse LAG3 having the amino acid sequence of SEQ ID NO: 2. In a certain embodiment, the heavy and light chain variable regions of an anti-LAG3 antibody are selected from the heavy and light chain variable regions described with respect to the first binding site that binds to LAG3 of the bispecific molecule. In another embodiment, an anti-LAG3 antibody is provided that competes with these antibodies for the binding to LAG3.

The anti-LAG3 antibody may be derived from any of animal species such as mice, rats, rabbits, and goats, and may be an antibody derived from a human or a humanized antibody. The anti-LAG3 antibody may be an antibody of any immunoglobulin class. Examples of the immunoglobulin class include IgA, IgD, IgE, IgG, and IgM, and examples of the subclass (isotype) of the immunoglobulin class include IgG1, IgG2, IgG3, IgG4, IgA1, and IgA2. In a certain embodiment, the anti-LAG3 antibody is of the IgG subclass, e.g., the IgG1 or IgG4 subclass.

The fragment of the anti-LAG3 antibody contains a part of the anti-LAG3 antibody as a component, and includes a molecule that retains binding to LAG3, for example, heavy chain and light chain variable regions (V_{H} and V_{L}) of an anti-LAG3 antibody, F(ab')₂, Fab', Fab, Fv, disulphide-linked FV (sdFv), Single-Chain FV (scFV), Fab3, diabody, triabody, tetrabody, minibody, Bis-scFv, (scFv)₂-Fc, intact-IgG, sc(Fv)₂, covalent diabody, (FvCys)₂, F(ab'-zipper)₂, (Fv-zipper)₂, three-chain antibody, mAb², tandem scFv, scDiabody, FabFv, Fab'Fv, FabdsFv, Fab-scFv, Fab-dsscFv, Fab-(dsscFv)2, diFab, diFab', scFv-Fc, tandem scFv-Fc, scDiabody-Fc, scDiabody-CH3, Ig-scFv, and scFv-Ig.

The present disclosure provides, for example, the following embodiments.
[1] An immunosuppressant containing a bispecific molecule including a first binding site that specifically binds to LAG3 and a second binding site that specifically binds to CD3 or CD8.
[2] The immunosuppressant according to [1] above, wherein the bispecific molecule allows binding of LAG3 to an MHC class II molecule.
[3] The immunosuppressant according to [1] or [2] above, wherein the first binding site binds to a portion that is included in a D1 region of LAG3 and is not included in an extra loop region of LAG3.
[4] The immunosuppressant according to any one of [1] to [3] above, wherein the first binding site binds to a region corresponding to a region ranging from serine at position 23 to serine at position 69 of mouse LAG3 having the amino acid sequence of SEQ ID NO: 2.
[5] The immunosuppressant according to any one of [1] to [4] above, wherein the first binding site binds to a region including an amino acid/amino acids corresponding to asparagine at position 54, phenylalanine at position 55, valine at position 61, and/or isoleucine at position 62 of mouse LAG3 having the amino acid sequence of SEQ ID NO: 2.
[6] The immunosuppressant according to any one of [1] to [5] above, wherein the first binding site includes a heavy chain variable region and a light chain variable region of an anti-LAG3 antibody.
[7] The immunosuppressant according to [6] above,
   wherein the first binding site includes:
   a heavy chain variable region that includes a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
   a light chain variable region that includes a light chain CDR1 including an amino acid sequence of SEQ ID NO: 12, a light chain CDR2 including an amino acid sequence of SEQ ID NO: 13, and a light chain CDR3 including an amino acid sequence of SEQ ID NO: 14.
[8] The immunosuppressant according to [6] or [7] above,
   wherein the first binding site includes:
   a heavy chain variable region that includes an amino acid sequence having an identity of 90% or more with the amino acid sequence of SEQ ID NO: 7; and
   a light chain variable region that includes an amino acid sequence having an identity of 90% or more with the amino acid sequence of SEQ ID NO: 8.
[9] The immunosuppressant according to any one of [1] to [6] above,
   wherein the first binding site competes, for the binding to LAG3, with an anti-LAG3 antibody that includes:
   (i) a heavy chain variable region including a heavy chain CDR1 including the amino acid sequence of SEQ ID NO: 9, a heavy chain CDR2 including the amino acid sequence of SEQ ID NO: 10, and a heavy chain CDR3 including the amino acid sequence of SEQ ID NO: 11; and
      a light chain variable region including a light chain CDR1 including the amino acid sequence of SEQ ID NO: 12, a light chain CDR2 including the amino acid sequence of SEQ ID NO: 13 and a light chain CDR3 including the amino acid sequence of SEQ ID NO: 14, or
   (ii) a heavy chain variable region including the amino acid sequence of SEQ ID NO: 7, and a light chain variable region including the amino acid sequence of SEQ ID NO: 8.
[10] The immunosuppressant according to any one of [1] to [6] above,
   wherein the binding of the first binding site to LAG3 is subjected to competition with an anti-LAG3 antibody that includes:
   (i) a heavy chain variable region including a heavy chain CDR1 including the amino acid sequence of SEQ ID NO: 9, a heavy chain CDR2 including the amino acid sequence of SEQ ID NO: 10, and a heavy chain CDR3 including the amino acid sequence of SEQ ID NO: 11; and
      a light chain variable region including a light chain CDR1 including the amino acid sequence of SEQ ID NO: 12, a light chain CDR2 including the amino acid sequence of SEQ ID NO: 13 and a light chain CDR3 including the amino acid sequence of SEQ ID NO: 14, or
   (ii) a heavy chain variable region including the amino acid sequence of SEQ ID NO: 7, and a light chain variable region including the amino acid sequence of SEQ ID NO: 8.
[11] The immunosuppressant according to any one [1] to [10] above, wherein the second binding site specifically binds to CD3.
[12] The immunosuppressant according to [11] above, wherein the second binding site specifically binds to CD3ε.
[13] The immunosuppressant according to [11] or [12] above, wherein the second binding site includes a heavy chain variable region and a light chain variable region of an anti-CD3 antibody.
[14] The immunosuppressant according to [13] above,
   wherein the second binding site includes:
   a heavy chain variable region that includes a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
   a light chain variable region that includes a light chain CDR1 including an amino acid sequence of SEQ ID NO: 20, a light chain CDR2 including an amino acid sequence of SEQ ID NO: 21, and a light chain CDR3 including an amino acid sequence of SEQ ID NO: 22.
[15] The immunosuppressant according to [13] or [14] above,
   wherein the second binding site includes:
   a heavy chain variable region that includes an amino acid sequence having an identity of 90% or more with the amino acid sequence of SEQ ID NO: 15; and
   a light chain variable region that includes an amino acid sequence having an identity of 90% or more with the amino acid sequence of SEQ ID NO: 16.
[16] The immunosuppressant according to any one of [11] to [13] above,
   wherein the second binding site competes, for the binding to CD3, with an anti-CD3 antibody that includes:
   (i) a heavy chain variable region including a heavy chain CDR1 including the amino acid sequence of SEQ ID NO: 17, a heavy chain CDR2 including the amino acid sequence of SEQ ID NO: 18, and a heavy chain CDR3 including the amino acid sequence of SEQ ID NO: 19; and
      a light chain variable region including a light chain CDR1 including the amino acid sequence of SEQ ID NO: 20, a light chain CDR2 including the amino acid sequence of SEQ ID NO: 21 and a light chain CDR3 including the amino acid sequence of SEQ ID NO: 22, or
   (ii) a heavy chain variable region including the amino acid sequence of SEQ ID NO: 15, and a light chain variable region including the amino acid sequence of SEQ ID NO: 16.
[17] The immunosuppressant according to any one of [11] to [13] above,
   wherein the binding of the second binding site to CD3 is subjected to competition with an anti-CD3 antibody that includes:
   (i) a heavy chain variable region including a heavy chain CDR1 including the amino acid sequence of SEQ ID NO: 17, a heavy chain CDR2 including the amino acid sequence of SEQ ID NO: 18, and a heavy chain CDR3 including the amino acid sequence of SEQ ID NO: 19; and
      a light chain variable region including a light chain CDR1 including the amino acid sequence of SEQ ID NO: 20, a light chain CDR2 including the amino acid sequence of SEQ ID NO: 21 and a light chain CDR3 including the amino acid sequence of SEQ ID NO: 22, or
   (ii) a heavy chain variable region including the amino acid sequence of SEQ ID NO: 15, and a light chain variable region including the amino acid sequence of SEQ ID NO: 16.
[18] The immunosuppressant according to any one of [1] to [10] above, wherein the second binding site specifically binds to CD8.
[19] The immunosuppressant according to [18] above, wherein the second binding site specifically binds to CD8α.
[20] The immunosuppressant according to [18] or [19] above, wherein the second binding site includes a heavy chain variable region and a light chain variable region of an anti-CD8 antibody.
[21] The immunosuppressant according to [20] above,
   wherein the second binding site includes:
   a heavy chain variable region that includes a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
   a light chain variable region that includes a light chain CDR1 including an amino acid sequence of SEQ ID NO: 28, a light chain CDR2 including an amino acid sequence of SEQ ID NO: 29, and a light chain CDR3 including an amino acid sequence of SEQ ID NO: 30.
[22] The immunosuppressant according to [20] or [21] above,
   wherein the second binding site includes:
   a heavy chain variable region that includes an amino acid sequence having an identity of 90% or more with the amino acid sequence of SEQ ID NO: 23; and
   a light chain variable region that includes an amino acid sequence having an identity of 90% or more with the amino acid sequence of SEQ ID NO: 24.
[23] The immunosuppressant according to any one of [18] to [20] above,
   wherein the second binding site competes, for the binding to CD8, with an anti-CD8 antibody that includes:
   (i) a heavy chain variable region including a heavy chain CDR1 including the amino acid sequence of SEQ ID NO: 25, a heavy chain CDR2 including the amino acid sequence of SEQ ID NO: 26, and a heavy chain CDR3 including the amino acid sequence of SEQ ID NO: 27; and
      a light chain variable region including a light chain CDR1 including the amino acid sequence of SEQ ID NO: 28, a light chain CDR2 including the amino acid sequence of SEQ ID NO: 29 and a light chain CDR3 including the amino acid sequence of SEQ ID NO: 30, or
   (ii) a heavy chain variable region including the amino acid sequence of SEQ ID NO: 23, and a light chain variable region including the amino acid sequence of SEQ ID NO: 24.
[24] The immunosuppressant according to any one of [18] to [20] above,
   wherein the binding of the second binding site to CD8 is subjected to competition with an anti-CD8 antibody that includes:
   (i) a heavy chain variable region including a heavy chain CDR1 including the amino acid sequence of SEQ ID NO: 25, a heavy chain CDR2 including the amino acid sequence of SEQ ID NO: 26, and a heavy chain CDR3 including the amino acid sequence of SEQ ID NO: 27; and
      a light chain variable region including a light chain CDR1 including the amino acid sequence of SEQ ID NO: 28, a light chain CDR2 including the amino acid sequence of SEQ ID NO: 29 and a light chain CDR3 including the amino acid sequence of SEQ ID NO: 30, or
   (ii) a heavy chain variable region including the amino acid sequence of SEQ ID NO: 23, and a light chain variable region including the amino acid sequence of SEQ ID NO: 24.
[25] The immunosuppressant according to any one of [1] to [24] above, wherein the bispecific molecule is in any form of bispecific antibody, diabody, tandem scFv, scDiabody, FabFv, Fab'Fv, FabdsFv, Fab-scFv, Fab-dsscFv, Fab-(dsscFv)2, diFab, diFab', scFv-Fc, tandem scFv-Fc, scDiabody-Fc, scDiabody-CH3, Ig-scFv, and scFv-Ig.
[26] The immunosuppressant according to any one of [1] to [25] above, wherein the bispecific molecule is in any form of diabody, tandem scFv, and scDiabody (preferably, in the form of scDiabody).
[27] The immunosuppressant according to any one of [1] to [25] above, wherein the bispecific molecule is in the form of bispecific antibody (preferably in the form of bispecific monoclonal antibody).
[28] The immunosuppressant according to any one of [1] to [27] above, for prevention and/or treatment of an autoimmune disease, an allergic disease, or a graft-versus-host disease.
[29] A method for preventing and/or treating an autoimmune disease, an allergic disease, or a graft-versus-host disease, the method including:
   administering an effective amount of the immunosuppressant according to any one of [1] to [27] above, to a subject in need thereof.
[30] The immunosuppressant according to any one of [1] to [27] above, for use in prevention and/or treatment of an autoimmune disease, an allergic disease, or a graft-versus-host disease.
[31] A use of the immunosuppressant according to any one of [1] to [27] above for production of a prophylactic and/or therapeutic agent for an autoimmune disease, an allergic disease, or a graft-versus-host disease.
[32] A bispecific molecule including a first binding site that specifically binds to LAG3 and a second binding site that specifically binds to CD3 or CD8.
[33] The bispecific molecule according to [32] above, allowing binding of LAG3 to an MHC class II molecule.
[34] The bispecific molecule according to [32] or [33] above, wherein the first binding site binds to a portion that is included in a D1 region of LAG3 and is not included in an extra loop region of LAG3.
[35] The bispecific molecule according to any one of [32] to [34] above, wherein the first binding site binds to a region corresponding to a region ranging from serine at position 23 to serine at position 69 of mouse LAG3 having the amino acid sequence of SEQ ID NO: 2.
[36] The bispecific molecule according to any one of [32] to [35] above, wherein the first binding site binds to a region including an amino acid/amino acids corresponding to asparagine at position 54, phenylalanine at position 55, valine at position 61, and/or isoleucine at position 62 of mouse LAG3 having the amino acid sequence of SEQ ID NO: 2.
[37] The bispecific molecule according to any one of [32] to [36] above, wherein the first binding site includes a heavy chain variable region and a light chain variable region of an anti-LAG3 antibody.
[38] The bispecific molecule according to [37] above,
   wherein the first binding site includes:
   a heavy chain variable region that includes a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
   a light chain variable region that includes a light chain CDR1 including an amino acid sequence of SEQ ID NO: 12, a light chain CDR2 including an amino acid sequence of SEQ ID NO: 13, and a light chain CDR3 including an amino acid sequence of SEQ ID NO:14.
[39] The bispecific molecule according to [37] or [38] above,
   wherein the first binding site includes:
   a heavy chain variable region that includes an amino acid sequence having an identity of 90% or more with the amino acid sequence of SEQ ID NO: 7; and
   a light chain variable region that includes an amino acid sequence having an identity of 90% or more with the amino acid sequence of SEQ ID NO: 8.
[40] The bispecific molecule according to any one of [32] to [37] above,
   wherein the first binding site competes, for the binding to LAG3, with an anti-LAG3 antibody that includes:
   (i) a heavy chain variable region including a heavy chain CDR1 including the amino acid sequence of SEQ ID NO: 9, a heavy chain CDR2 including the amino acid sequence of SEQ ID NO: 10, and a heavy chain CDR3 including the amino acid sequence of SEQ ID NO: 11; and
      a light chain variable region including a light chain CDR1 including the amino acid sequence of SEQ ID NO: 12, a light chain CDR2 including the amino acid sequence of SEQ ID NO: 13 and a light chain CDR3 including the amino acid sequence of SEQ ID NO: 14, or
   (ii) a heavy chain variable region including the amino acid sequence of SEQ ID NO: 7, and a light chain variable region including the amino acid sequence of SEQ ID NO: 8.
[41] The bispecific molecule according to any one of [32] to [37] above,
   wherein the binding of the first binding site to LAG3 is subjected to competition with an anti-LAG3 antibody that includes:
   (i) a heavy chain variable region including a heavy chain CDR1 including the amino acid sequence of SEQ ID NO: 9, a heavy chain CDR2 including the amino acid sequence of SEQ ID NO: 10, and a heavy chain CDR3 including the amino acid sequence of SEQ ID NO: 11; and
      a light chain variable region including a light chain CDR1 including the amino acid sequence of SEQ ID NO: 12, a light chain CDR2 including the amino acid sequence of SEQ ID NO: 13 and a light chain CDR3 including the amino acid sequence of SEQ ID NO: 14, or
   (ii) a heavy chain variable region including the amino acid sequence of SEQ ID NO: 7, and a light chain variable region including the amino acid sequence of SEQ ID NO: 8.
[42] The bispecific molecule according to any one of [32] to [41] above, wherein the second binding site specifically binds to CD3.
[43] The bispecific molecule according to [42], wherein the second binding site specifically binds to CD3ε.
[44] The bispecific molecule according to [42] or [43] above, wherein the second binding site includes a heavy chain variable region and a light chain variable region of an anti-CD3 antibody.
[45] The bispecific molecule according to [44] above,
   wherein the second binding site includes:
   a heavy chain variable region that includes a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
   a light chain variable region that includes a light chain CDR1 including an amino acid sequence of SEQ ID NO: 20, a light chain CDR2 including an amino acid sequence of SEQ ID NO: 21, and a light chain CDR3 including an amino acid sequence of SEQ ID NO: 22.
[46] The bispecific molecule according to [44] or [45] above,
   wherein the second binding site includes:
   a heavy chain variable region that includes an amino acid sequence having an identity of 90% or more with the amino acid sequence of SEQ ID NO: 15; and
   a light chain variable region that includes an amino acid sequence having an identity of 90% or more with the amino acid sequence of SEQ ID NO: 16.
[47] The bispecific molecule according to any one of [42] to [44] above,
   wherein the second binding site competes, for the binding to CD3, with an anti-CD3 antibody that includes:
   (i) a heavy chain variable region including a heavy chain CDR1 including the amino acid sequence of SEQ ID NO: 17, a heavy chain CDR2 including the amino acid sequence of SEQ ID NO: 18, and a heavy chain CDR3 including the amino acid sequence of SEQ ID NO: 19; and
      a light chain variable region including a light chain CDR1 including the amino acid sequence of SEQ ID NO: 20, a light chain CDR2 including the amino acid sequence of SEQ ID NO: 21 and a light chain CDR3 including the amino acid sequence of SEQ ID NO: 22, or
   (ii) a heavy chain variable region including the amino acid sequence of SEQ ID NO: 15, and a light chain variable region including the amino acid sequence of SEQ ID NO: 16.
[48] The bispecific molecule according to any one of [42] to [44] above,
   wherein the binding of the second binding site to CD3 is subjected to competition with an anti-CD3 antibody that includes:
   (i) a heavy chain variable region including a heavy chain CDR1 including the amino acid sequence of SEQ ID NO: 17, a heavy chain CDR2 including the amino acid sequence of SEQ ID NO: 18, and a heavy chain CDR3 including the amino acid sequence of SEQ ID NO: 19; and
      a light chain variable region including a light chain CDR1 including the amino acid sequence of SEQ ID NO: 20, a light chain CDR2 including the amino acid sequence of SEQ ID NO: 21 and a light chain CDR3 including the amino acid sequence of SEQ ID NO: 22, or
   (ii) a heavy chain variable region including the amino acid sequence of SEQ ID NO: 15, and a light chain variable region including the amino acid sequence of SEQ ID NO: 16.
[49] The bispecific molecule according to any one of [32] to [41] above, wherein the second binding site specifically binds to CD8.
[50] The bispecific molecule according to [49], wherein the second binding site specifically binds to CD8α.
[51] The bispecific molecule according to [49] or [50] above, wherein the second binding site includes a heavy chain variable region and a light chain variable region of an anti-CD8 antibody.
[52] The bispecific molecule according to [51] above,
   wherein the second binding site includes:
   a heavy chain variable region that includes a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
   a light chain variable region that includes a light chain CDR1 including an amino acid sequence of SEQ ID NO: 28, a light chain CDR2 including an amino acid sequence of SEQ ID NO: 29, and a light chain CDR3 including an amino acid sequence of SEQ ID NO: 30.
[53] The bispecific molecule according to [51] or [52] above,
   wherein the second binding site includes:
   a heavy chain variable region that includes an amino acid sequence having an identity of 90% or more with the amino acid sequence of SEQ ID NO: 23; and
   a light chain variable region that includes an amino acid sequence having an identity of 90% or more with the amino acid sequence of SEQ ID NO: 24.
[54] The bispecific molecule according to any one of [49] to [51] above,
   wherein the second binding site competes, for the binding to CD8, with an anti-CD8 antibody that includes:
   (i) a heavy chain variable region including a heavy chain CDR1 including the amino acid sequence of SEQ ID NO: 25, a heavy chain CDR2 including the amino acid sequence of SEQ ID NO: 26, and a heavy chain CDR3 including the amino acid sequence of SEQ ID NO: 27; and
      a light chain variable region including a light chain CDR1 including the amino acid sequence of SEQ ID NO: 28, a light chain CDR2 including the amino acid sequence of SEQ ID NO: 29 and a light chain CDR3 including the amino acid sequence of SEQ ID NO: 30, or
   (ii) a heavy chain variable region including the amino acid sequence of SEQ ID NO: 23, and a light chain variable region including the amino acid sequence of SEQ ID NO: 24.
[55] The bispecific molecule according to any one of [49] to [51] above,
   wherein the binding of the second binding site to CD8 is subjected to competition with an anti-CD8 antibody that includes:
   (i) a heavy chain variable region including a heavy chain CDR1 including the amino acid sequence of SEQ ID NO: 25, a heavy chain CDR2 including the amino acid sequence of SEQ ID NO: 26, and a heavy chain CDR3 including the amino acid sequence of SEQ ID NO: 27; and
      a light chain variable region including a light chain CDR1 including the amino acid sequence of SEQ ID NO: 28, a light chain CDR2 including the amino acid sequence of SEQ ID NO: 29 and a light chain CDR3 including the amino acid sequence of SEQ ID NO: 30, or
   (ii) a heavy chain variable region including the amino acid sequence of SEQ ID NO: 23, and a light chain variable region including the amino acid sequence of SEQ ID NO: 24.
[56] The bispecific molecule according to any one of [32] to [55] above, wherein the bispecific molecule is in any form of bispecific antibody, diabody, tandem scFv, scDiabody, FabFv, Fab'Fv, FabdsFv, Fab-scFv, Fab-dsscFv, Fab-(dsscFv)2, diFab, diFab', scFv-Fc, tandem scFv-Fc, scDiabody-Fc, scDiabody-CH3, Ig-scFv, and scFv-Ig.
[57] The bispecific molecule according to any one of [32] to [56] above, wherein the bispecific molecule is in any form of diabody, tandem scFv, and scDiabody (preferably, in the form of scDiabody).
[58] The bispecific molecule according to any one of [32] to [56] above, wherein the bispecific molecule is in the form of bispecific antibody (preferably in the form of bispecific monoclonal antibody).
[59] An immunosuppressant containing the bispecific molecule according to any one of [32] to [58] above as an active ingredient.
[60] The immunosuppressant according to [59] above, further containing a pharmaceutically acceptable carrier.
[61] A method for suppressing immunity, the method including administering an effective amount of the bispecific molecule according to any one of [32] to [58] above, to a subject in need thereof.
[62] The bispecific molecule according to any one of [32] to [58] above, for use in immunosuppression.
[63] A use of the bispecific molecule according to any one of [32] to [58] above, for the production of an immunosuppressant.
[64] A prophylactic and/or therapeutic agent for a disease characterized by enhanced immunity, the prophylactic and/or therapeutic agent containing the bispecific molecule according to any one of [32] to [58] above as an active ingredient.
[65] The prophylactic and/or therapeutic agent according to [64] above, further containing a pharmaceutically acceptable carrier.
[66] A method for preventing and/or treating a disease characterized by enhanced immunity, the method including administering an effective amount of the bispecific molecule according to any one of [32] to [58] above, to a subject in need thereof.
[67] The bispecific molecule according to any one of [32] to [58] above, for use in the prevention and/or treatment of a disease characterized by enhanced immunity.
[68] A use of the bispecific molecule according to any one of [32] to [58] above, for the production of a prophylactic and/or therapeutic agent for a disease characterized by enhanced immunity.
[69] The prophylactic and/or therapeutic agent according to [64] or [65] above, the method according to [66] above, the bispecific molecule according to [67] above, or the use according to [68] above,
   wherein the disease characterized by enhanced immunity is an autoimmune disease, an allergic disease, or a graft-versus-host disease.
[70] The prophylactic and/or therapeutic agent, method, bispecific molecule, or use according to [69] above, wherein the disease characterized by enhanced immunity is an autoimmune disease.
[71] The prophylactic and/or therapeutic agent, method, bispecific molecule, or use according to [70] above, the autoimmune disease is selected from the group consisting of Behcet's disease, systemic lupus erythematosus, multiple sclerosis, scleroderma, polymyositis, dermatomyositis, periarteritis nodosa, aortitis syndrome, malignant rheumatoid arthritis, rheumatoid arthritis, juvenile idiopathic arthritis, Wegener's granulomatosis, mixed connective tissue disease, Sjogren's syndrome, Adult-onset Still's disease, allergic granulomatous angiitis, hypersensitivity vasculitis, Cogan's syndrome, RS3PE syndrome, temporal arteritis, polymyalgia rheumatica, fibromyalgia, antiphospholipid antibody syndrome, eosinophilic fasciitis, IgG4-related diseases, Guillain-Barre syndrome, myasthenia gravis, chronic atrophic gastritis, autoimmune hepatitis, primary biliary cirrhosis, aortitis syndrome, Goodpasture syndrome, rapidly progressive glomerulonephritis, megaloblastic anemia, autoimmune hemolytic anemia, autoimmune neutropenia, idiopathic thrombocytopenic purpura, Basedow's disease, Hashimoto disease, autoimmune adrenal insufficiency, primary hypothyroidism, idiopathic Addison disease, type I diabetes, slowly progressive type I diabetes, chronic discoid lupus erythematosus, circumscribed scleroderma, psoriasis, psoriatic arthritis, pemphigus, pemphigoid, gestational herpes, linear IgA bullous dermatosis, acquired epidermolysis bullosa, alopecia areata, white spots, vitiligo vulgaris, atopic dermatitis, neuromyelitis optica, Chronic inflammatory demyelinating polyneuropathy, sarcoidosis, bullous pemphigoid, giant cell arteritis, amyotrophic lateral sclerosis, eosinophilic granulomatosis with polyangiitis, Harada disease, autoimmune optic neuropathy, idiopathic azoospermia, habitual abortion, inflammatory bowel disease, and celiac disease.
[72] The prophylactic and/or therapeutic agent, method, bispecific molecule, or use according to [70] or [71] above, wherein the autoimmune disease is type I diabetes, multiple sclerosis, systemic lupus erythematosus, or rheumatoid arthritis.
[73] The prophylactic and/or therapeutic agent, method, bispecific molecule or use according to any one of [70] to [72] above, wherein the autoimmune disease is multiple sclerosis.
[74] The prophylactic and/or therapeutic agent, method, bispecific molecule or use according to [73] above, wherein the multiple sclerosis is systemic scleroderma or progressive systemic sclerosis.
[75] A polynucleotide encoding the bispecific molecule according to any one of [32] to [58] above.
[76] An expression vector including the polynucleotide according to [75] above.
[77] A host cell containing the polynucleotide according to [75] above or the expression vector according to [76] above.
[78] An anti-LAG3 antibody or a fragment thereof binding to a region including an amino acid/amino acids corresponding to asparagine at position 54, phenylalanine at position 55, valine at position 61, and/or isoleucine at position 62 of mouse LAG3 having the amino acid sequence of SEQ ID NO: 2.
[79] An anti-LAG3 antibody or a fragment thereof including:
   a heavy chain variable region that includes a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11, and
   a light chain variable region that includes a light chain CDR1 including an amino acid sequence of SEQ ID NO: 12, a light chain CDR2 including an amino acid sequence of SEQ ID NO: 13, and a light chain CDR3 including an amino acid sequence of SEQ ID NO: 14.
[80] The anti-LAG3 antibody or a fragment thereof according to [79] above,
   wherein the anti-LAG3 antibody includes:
   a heavy chain variable region that includes an amino acid sequence having an identity of 90% or more with the amino acid sequence of SEQ ID NO: 7; and
   a light chain variable region that includes an amino acid sequence having an identity of 90% or more with the amino acid sequence of SEQ ID NO: 8.
[81] An anti-LAG3 antibody or a fragment thereof that competes with the antibody of [79] or [80] above for the binding to LAG3.

All references cited herein are hereby incorporated by reference.

All of the above description is non-limiting and can be modified without departing from the scope of the invention as defined in the appended claims. In addition, the examples below are all non-limiting examples and are provided solely to illustrate the invention.

### EXAMPLES

### Example 1: Production of bispecific molecules 2C11xTKB58 and TKB58x2C11 binding to LAG3 and CD3ε

A nucleic acid encoding the heavy and light chain variable regions of an anti-mouse LAG3 antibody (TKB58) and an anti-mouse CD3ε antibody (2C11) was synthesized, amplified by PCR, and was cloned into an expression plasmid vector produced by modifying pEBMulti-Neo (Wako) or pSecTag2/Hygro (Thermo Fisher Scientific), whereby expression plasmids of bispecific molecules 2C11xTKB58 (SEQ ID NO: 31) and TKB58x2C11 (SEQ ID NO: 32) recognizing mouse LAG3 and mouse CD3ε were produced. The expression plasmid was transfected into PlatE cells using Avalanche-Omni Transfection Reagent (EZ Biosystems), and the culture supernatant was collected after 48 hours. BW5147, DO11.10, and D011.10-mLAG3 cells were stained using the culture supernatant.

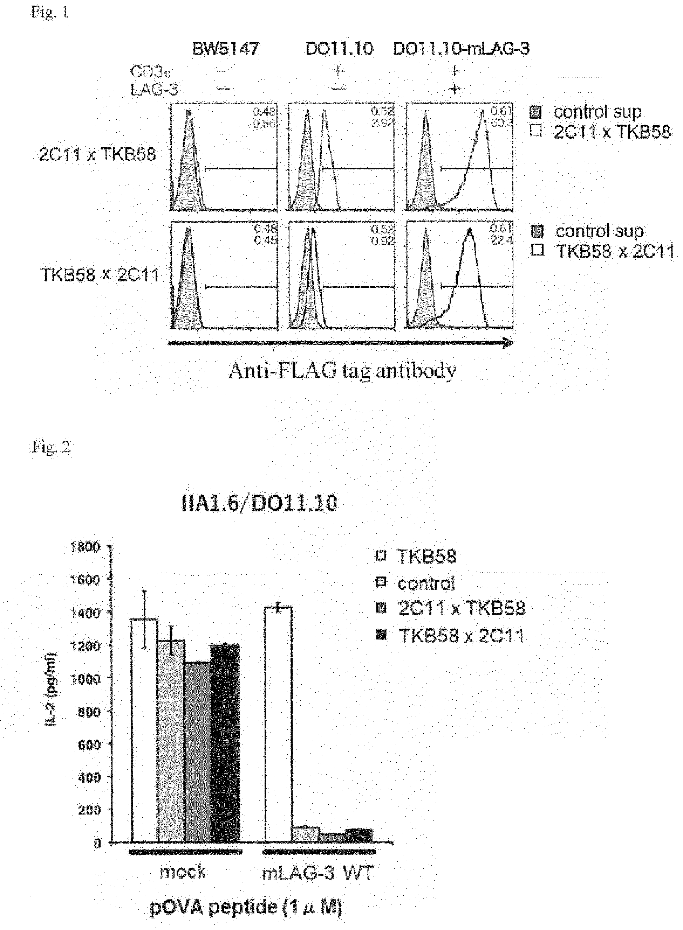

The results are shown in Fig. 1. Binding to DO11.10 cells expressing mouse CD3ε but not mouse LAG3 was confirmed, from which it was confirmed that the bispecific molecule had a binding ability to mouse CD3ε. In addition, as compared with DO11.10 cells, the bispecific molecule strongly bound to D011.10-mLAG3 cells in which mouse LAG3 was forcibly expressed, from which it was also confirmed that the bispecific molecule had a binding ability to mouse LAG3. On the other hand, no binding was observed to BW5147 cells that do not express mouse CD3ε and mouse LAG3, from which it was confirmed that the binding is specific to both molecules. A stronger binding was observed with 2C11xTKB58, as compared to TKB58x2C11.

### Example 2: Bispecific molecules 2C11xTKB58 and TKB58x2C11 suppress antigen-specific activation of T cells in LAG3 dependent manner

### Example 2-1: Experiments with antigen-presenting cells strongly inducing suppression by LAG3

DO11.10 cells are known to recognize an OVA-derived peptide (pOVA323-339, ISQAVHAAHAEINEAGR) presented on the mouse MHC class II molecule I-A^{d} and produce IL-2 depending on the amount of an antigenic peptide. When IIA1.6 cells expressing I-A^{d} were pulsed with pOVA323-339 to stimulate DO11.10 cells (mock), production of IL-2 was observed, but neither TKB58x2C11 nor 2C11xTKB58 affected IL-2 production (Fig. 2). This confirmed that these bispecific molecules did not affect antigen-specific activation of T cells not expressing LAG3.

In addition, D011.10-mLAG3 cells (mLAG3 WT) obtained by causing mouse LAG3 to be forcibly expressed in DO11.10 cells were similarly antigen-stimulated (Fig. 2). The production of IL-2 was strongly suppressed in a LAG3 dependent manner. The addition of anti-mouse LAG3 antibody TKB58 inhibited the function of mouse LAG3 and restored the production of IL-2. On the other hand, the addition of TKB58x2C11 or 2C11xTKB58 did not inhibit the suppression of IL-2 production by LAG3.

### Example 2-2: Experiments with antigen-presenting cells that do not induce suppression by LAG3 too strongly

LAG3 selectively binds to a stable peptide MHCII complex and does not bind to an unstable peptide MHCII complex. When pOVA323-339 is pulsed into BW5147-mCD86/I-A^{d} cells obtained by forcibly expressing I-A^{d} in BW5147-mCD86 cells, pOVA323-339 does not bind to mouse LAG3, because pOVA323-339, with an unstable structure, is presented to I-A^{d}, resulting in that inhibition via mouse LAG3 hardly functions.

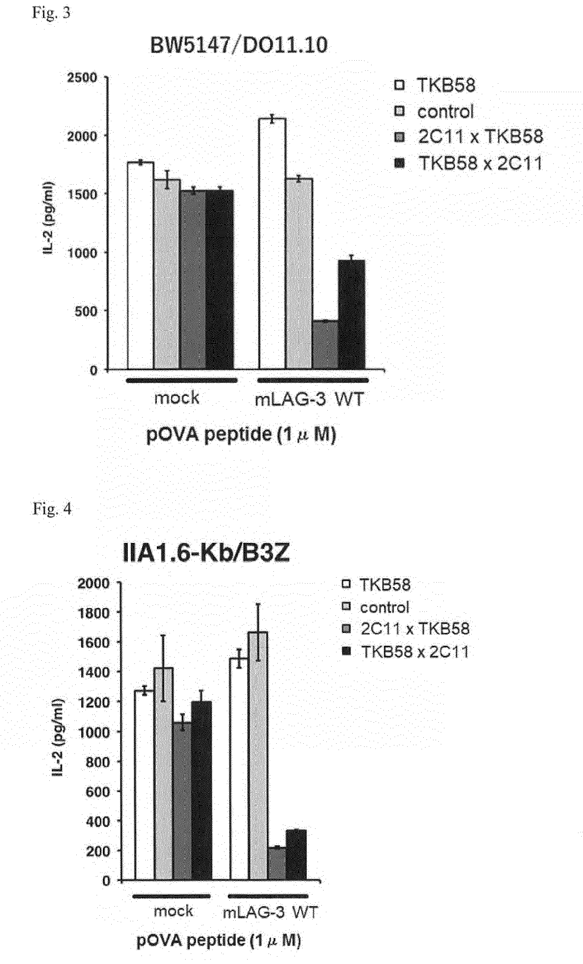

In this stimulation condition, when TKB58x2C11 or 2C11xTKB58 was added, the production of IL-2 was strongly suppressed only when DO11.10 cells expressing LAG3 (mLAG3WT) were used (Fig. 3). In the cell binding experiment (Fig. 1), similarly to the result that 2C11xTKB58 bound to the target cells more strongly than TKB58x2C11, 2C11xTKB58 showed a stronger inhibitory effect than TKB58x2C11.

### Example 2-3: Experiments with MHC class I-restricted cells in which LAG3 hardly exhibits an inhibitory effect

B3Z cells are known to recognize an OVA-derived peptide (pOVA257-264, SIINFEKL) presented on the mouse MHC class I molecule H-2K^{b} and produce IL-2 depending on the amount of an antigenic peptide. When IIA1.6 cells expressing H-2Kb were pulsed with the OVA peptide to stimulate B3Z cells (mock), production of IL-2 was observed, but the expression of mouse LAG3 (mLAG3WT) did not inhibit the production of IL-2 (Fig. 4). This is because LAG3 cannot exert the inhibitory function in the MHCI class I-restricted B3Z cells. The addition of TKB58x2C11 or 2C11xTKB58 strongly suppressed the production of IL-2 only when B3Z cells expressed mouse LAG3. These results indicate that TKB58x2C11 and 2C11xTKB58 suppress antigen-specific activation of LAG3 expressing cells, even in MHC class I-restricted CD8 positive cells.

### Example 2-4: Experiments with LAG3 mutant without ligand binding ability

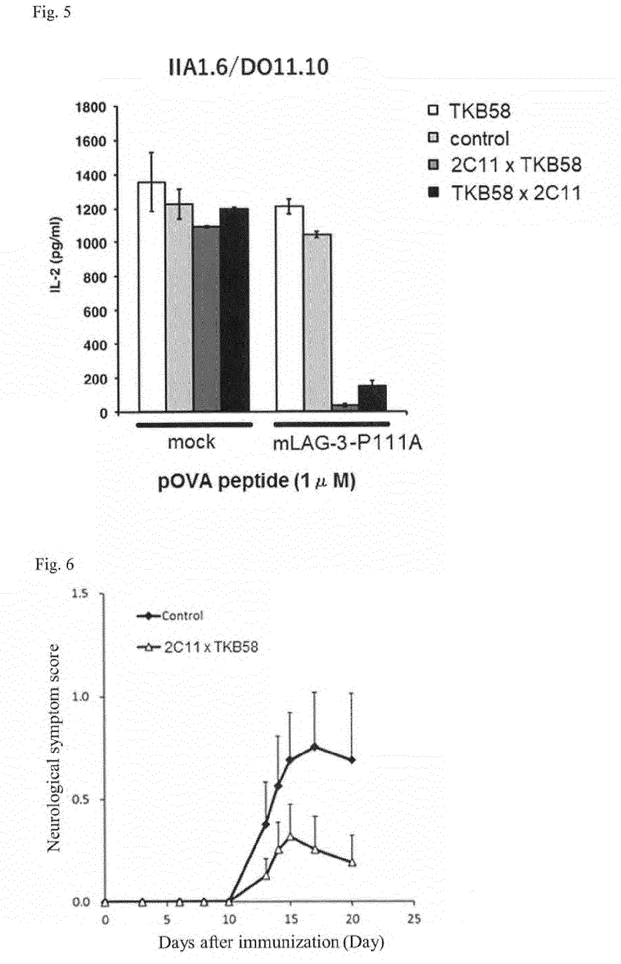

An amino acid mutant of LAG3, LAG3-P111A, lacks the ability to bind to pMHCII, and thus does not exert a T cell suppressive function even when it is antigen-stimulated. Using this mutant, the same experiment as in Example 2-1 was carried out. The results are shown in Fig. 5. When TKB58x2C11 or 2C11xTKB58 was added under this stimulation condition, the production of IL-2 was strongly suppressed in a LAG3-P111A-dependent manner. Therefore, it was revealed that TKB58x2C11 and 2C11xTKB58 suppress antigen-specific activation of LAG3-expressing cells even under conditions where LAG3 does not bind to pMHCII as a ligand. These results indicate that TKB58x2C11 and 2C11xTKB58 activate LAG3 and suppress TCR even in situations where LAG3 cannot bind to MHCII.

### Example 3: Evaluation of bispecific molecule using experimental autoimmune encephalomyelitis (EAE)

In vivo effects of 2C11xTKB58 were evaluated in an EAE model using C57BL/6 mice. Killed tuberculosis bacteria H37Ra (BD Biosciences, model number 231141) and incomplete Freund's adjuvant (BD Biosciences, model number 263910) were mixed so that complete Freund's adjuvant (CFA) containing 4 mg/mL of killed tuberculosis bacteria H37Ra was prepared. One mg/mL of MOG peptide (ANASPEC, model number AS-60130) and an equal amount of CFA were mixed so that an emulsion was prepared, and was used as an inducer of an EAE model. 200 µL of the inducer was subcutaneously administered to a base of the tail of a C57BL/6 mouse, and 200 µL of 1 µg/mL 100 day tussive toxin (SIGMA-ALDRICH, model number P7208) was intravenously administered on the day of immunization and day 2 of the immunization. C57BL/6 mice were then intraperitoneally administered with 2C11xTKB58 once daily at a dosage of 0.3 mg/kg each from day 6 to day 10 of immunization. After the day of immunization, the neurological symptoms were evaluated according to the method of Onuki et al. (Onuki M, et al., Microsc Res Tech 2001.; 52: 731 -9.), and the neurological symptom score was recorded (normal: score 0; tail relaxation: score 1; partial hind limb paralysis: score 2; postlimb paralysis: score 3; forelimb paralysis: score 4; moribund or dead: score 5). When a plurality of neurological symptoms were observed, a high value was adopted as the neurological symptom score of the evaluation date. The evaluation results (average value + standard error) are shown in Fig. 6. The bispecific molecule 2C11xTKB58 alleviated the symptoms of experimental autoimmune encephalomyelitis (EAE).

### Example 4: Experiments of binding of mouse LAG3 soluble protein to IIA1.6 cells in presence of bispecific molecule

A mouse LAG3 soluble protein was obtained as follows. cDNA fragments encoding D1 to D4 (LAG3-EC) of mouse LAG3 were amplified by PCR. A five-stranded coiled coil domain of a cartilage oligomer substrate protein (COMP) with a DYKDDDDK-tag, a TEV cleavage site, and a PA-tag was added to the C-terminus of LAG3-EC. The chimeric cDNA was cloned into an expression vector modified from pEBMulti-Neo (Wako). Plat-E cells were transfected with the plasmid using Avalanche-Omni Transfection Reagent (EZ Biosystems) and the culture supernatant was collected after 48 hours.

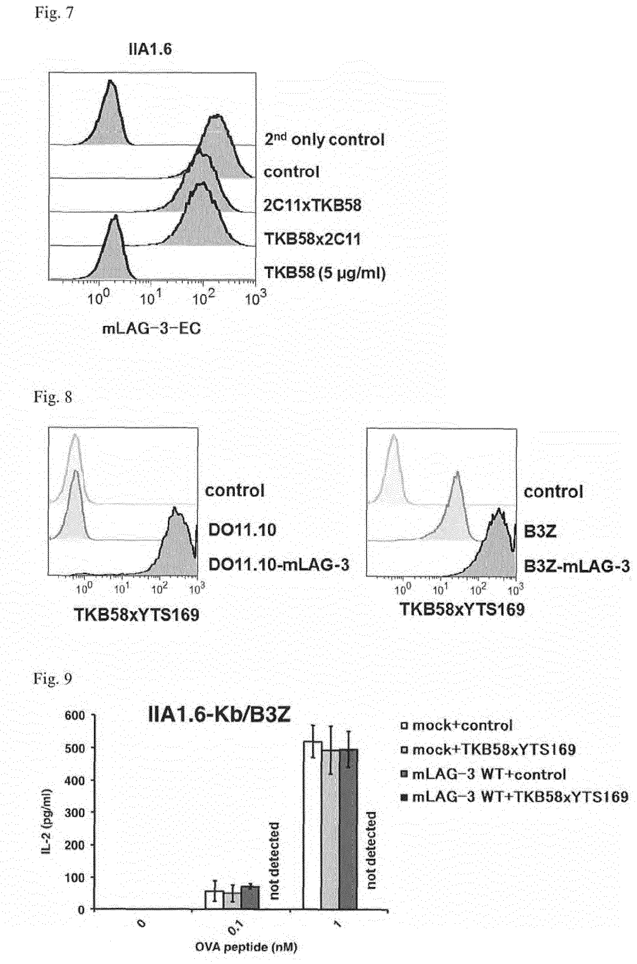

IIA1.6 cells expressing pMHCII, a ligand of mouse LAG3, were treated with TKB58x2C11, 2C11xTKB58, or a TKB58 antibody as a full-form anti-mouse LAG3 antibody, and thereafter, the cells were stained with a mouse LAG3 soluble protein. The results are shown in Fig. 7. The full-form TKB58 antibody completely inhibited the binding of the mouse LAG3 soluble protein to IIA1.6 cells, while TKB58x2C11 and 2C11xTKB58 did not.

### Example 5: Production of bispecific molecule TKB58xYTS169 binding to LAG3 and CD8

A nucleic acid encoding the heavy and light chain variable regions of an anti-mouse LAG3 antibody (TKB58) and an anti-mouse CD8 antibody (YTS169) was synthesized, amplified by PCR, and was cloned into an expression plasmid vector produced by modifying pEBMulti-Neo (Wako), whereby an expression plasmid of a bispecific molecule TKB58xYTS169 (SEQ ID NO: 33) recognizing mouse LAG3 and mouse CD8 was produced. The expression plasmid was transfected into PlatE cells using Avalanche-Omni Transfection Reagent (EZ Biosystems), and the culture supernatant was collected after 48 hours. DO11.10, DO11.10-mLAG3, B3Z, and B3Z-mLAG3 cells were stained using the culture supernatant.

The results are shown in Fig. 8. Binding to DO11.10 cells (expressing mouse LAG3 and not expressing mouse CD8) and B3Z cells (expressing mouse CD8 and not expressing mouse LAG3) was confirmed, which proves that TKB58xYTS169 had the binding ability to mouse CD8 and mouse LAG3. In addition, the bispecific molecule TKB58xYTS169 more strongly bound to B3Z-mLAG3 cells expressing mouse CD8 and mouse LAG3, which suggests that the bispecific molecule TKB58xYTS169 bound to mouse CD8 and mouse LAG3 on the same cells. On the other hand, binding to DO11.10 cells expressing neither of the foregoing molecules was not observed, with which it was confirmed that the binding was specific to both of the foregoing molecules.

### Example 6: Evaluation of bispecific molecule TKB58xYTS169 for antigen-specific activation of T cells

B3Z cells are known to recognize an OVA-derived peptide (pOVA257-264, SIINFEKL) presented on the mouse MHC class I molecule H-2K^{b} and produce IL-2 depending on the amount of an antigenic peptide. When IIA1.6 cells expressing H-2Kb were pulsed with pOVA257-264 to stimulate B3Z cells, production of IL-2 was observed, but the expression of mouse LAG3 did not inhibit the production of IL-2 (Fig. 9). This is because LAG3 cannot exert the inhibitory function in the MHCI class I-restricted B3Z cells, even if IIA1.6 cells express a ligand of LAG3. The addition of TKB58xYTS169 strongly suppressed the production of IL-2 only when B3Z cells expressed mouse LAG3. This revealed that TKB58xYTS169 suppressed antigen-specific activation of LAG3-expressing cells.

### Example 7: Binding of anti-mouse LAG3 antibody TKB58 to LAG3 mutants

### Example 7-1: Mouse LAG3 recognition region of anti-mouse LAG3 antibody TKB58

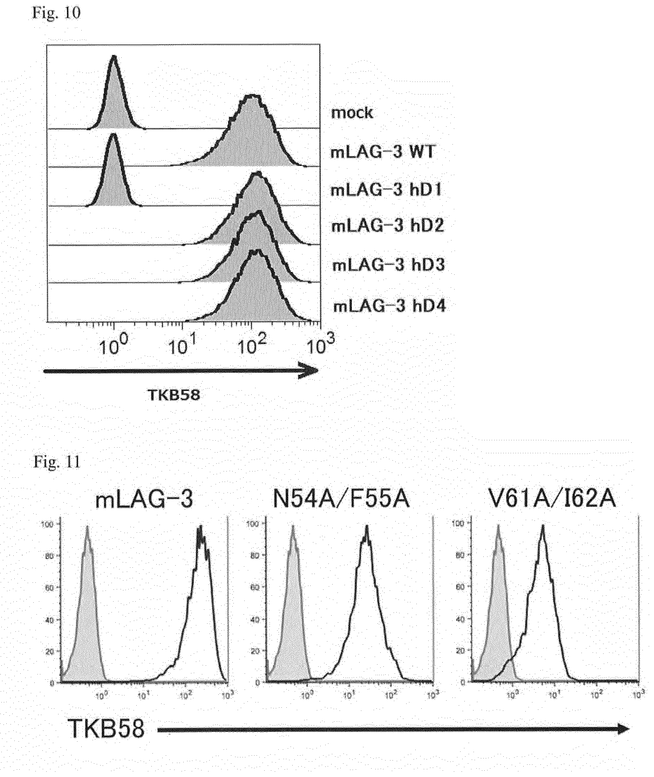

Mouse LAG3 has four Ig-like domains (D1, D2, D3, D4) in the extracellular region. Chimeric molecules in which the Ig-like domains were substituted with the corresponding human Ig-like domains, respectively, were produced and were forcibly expressed in DO11.10 cells. Briefly, each cDNA fragment was amplified by PCR and cloned into a retrovirus expression plasmid vector modified from pFB-ires-Neo (Agilent). Mouse and human LAG3 chimeric cDNAs were produced by overlap extension PCR. Plasmids were introduced into Plat-E cells (D'MEM, supplemented with high glucose (Gibco), 20% (v/v) FBS, 100 U/ml penicillin and 100 µg/ml streptomycin) using FuGENE HD (Promega). Using virus-containing supernatant, the genes were introduced into the target cells. Infected cells were selected by G418 (Wako), puromycin (Sigma-Aldrich), or cell sorting. The cells were stained using an anti-mouse LAG3 antibody (TKB58) and analyzed by flow cytometry. The results are shown in Fig. 10. TKB58 did not bind to the chimeric molecule in which mouse D1 was substituted with human D1, which reveals that TKB58 recognizes D1 of mouse LAG3.

### Example 7-2: Binding of anti-mouse LAG3 antibody TKB58 to various LAG3 mutants

Wild-type mouse LAG3, an N54A/F55A mutant, or a V61A/I62A mutant was forcibly expressed in DO11.10 T cells, and binding thereof to an anti-mouse LAG3 antibody (TKB58) was evaluated by flow cytometry. The results are shown in Fig. 11. The N54A/F55A mutant and the V61A/I62A mutant had reduced binding to TKB58.

### Example 7-3: Suppression of T cell function by LAG3 mutants

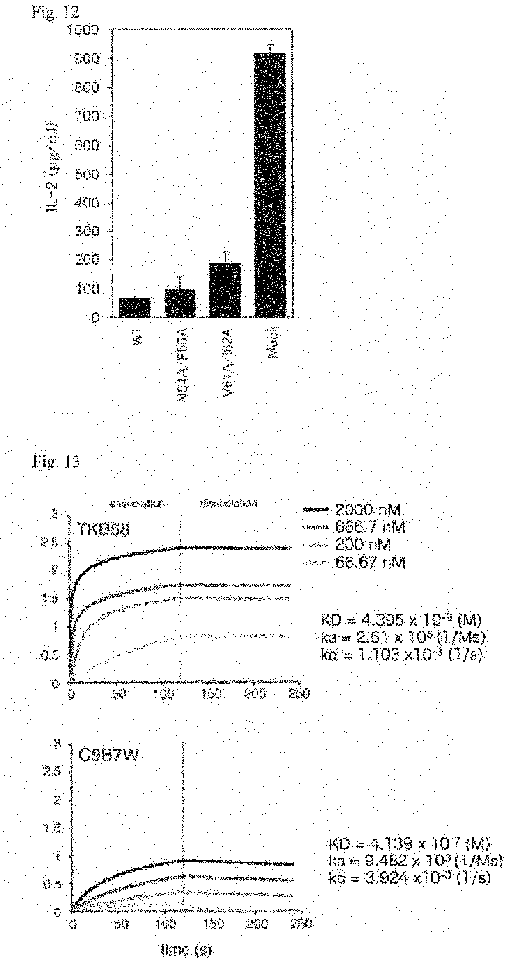

Wild-type mouse LAG3, an N54A/F55A mutant, or a V61A/I62A mutant was forcibly expressed in DO11.10 T cells. Using IIA1.6 cells pulsed with an OVA peptide, DO11.10 T cells were stimulated, and the concentration of IL-2 secreted into the culture supernatant was measured by ELISA. The results are shown in Fig. 12. Compared with DO11.10 mock cells in which mouse LAG3 was not expressed, the production of IL-2 by antigen stimulation was significantly reduced in DO11.10 T cells in which wild-type mouse LAG3, the N54A/F55A mutant, or the V61A/I62A mutant was forcibly expressed, with which it was confirmed that both mutants retained the activity of LAG3. Therefore, it was confirmed that the epitope of TKB58 includes a region that is not essential for the TCR suppressive function of LAG3.

### Example 8: Binding affinity of anti-mouse LAG3 antibody TKB58 to LAG3

The binding affinity of anti-mouse LAG3 antibodies (TKB58 and C9B7W) to a mouse LAG3 soluble protein was measured by biolayer interferometry. Briefly, cDNA fragments encoding D1 to D4 (LAG3-EC) of mouse LAG3 were amplified by PCR. A strep tag was added to the C-terminus of LAG3-EC. The chimeric cDNA was cloned into an expression vector modified from pEBMulti-Neo (Wako). Plat-E cells were transfected with the plasmid using Avalanche-Omni Transfection Reagent (EZ Biosystems) and the culture supernatant was collected after 48 hours. Monomeric mouse LAG3-EC (strep-tagged) was immobilized on a streptavidin-coated biosensor chip (Pall ForteBio) and binding of anti-mouse LAG3 antibodies at various concentrations was monitored with BLItz (Pall ForteBio). The chips were washed with PBS and the dissociation rates were analyzed. The binding rate constant (ka), the dissociation rate constant (kd), and the dissociation constant (kD) were calculated with BLItz Pro software. The results are shown in Fig. 13. In TKB58, as compared with a case in C9B7W, ka was about 26 times faster and kd was 3.6 times slower. As a result, the KD of TKB58 was 94 times lower than that of C9B7W, and was 4,395 nM.

### INDUSTRIAL APPLICABILITY

The bispecific molecule of the present disclosure is useful as an immunosuppressant, or is useful for preventing and/or treating an autoimmune disease, an allergic disease, or a graft-versus-host disease.

## Claims

1. An immunosuppressant comprising a bispecific molecule including a first binding site that specifically binds to LAG3 and a second binding site that specifically binds to CD3 or CD8.

2. The immunosuppressant according to claim 1, wherein the bispecific molecule allows binding of LAG3 to an MHC class II molecule.

3. The immunosuppressant according to claim 1 or 2, wherein the first binding site binds to a portion included in a D1 region of LAG3 and not included in an extra loop region of LAG3.

4. The immunosuppressant according to any one of claims 1 to 3, wherein the first binding site binds to a region including an amino acid/amino acids corresponding to asparagine at position 54, phenylalanine at position 55, valine at position 61, and/or isoleucine at position 62 of mouse LAG3 having an amino acid sequence of SEQ ID NO: 2.

5. The immunosuppressant according to any one of claims 1 to 4, wherein the first binding site includes a heavy chain variable region and a light chain variable region of an anti-LAG3 antibody.

6. The immunosuppressant according to claim 5,
wherein the first binding site includes:
a heavy chain variable region that includes a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
a light chain variable region that includes a light chain CDR1 including an amino acid sequence of SEQ ID NO: 12, a light chain CDR2 including an amino acid sequence of SEQ ID NO: 13, and a light chain CDR3 including an amino acid sequence of SEQ ID NO: 14.

7. The immunosuppressant according to claim 5 or 6,
wherein the first binding site includes:
a heavy chain variable region that includes an amino acid sequence having an identity of 90% or more with an amino acid sequence of SEQ ID NO: 7; and
a light chain variable region that includes an amino acid sequence having an identity of 90% or more with an amino acid sequence of SEQ ID NO: 8.

8. The immunosuppressant according to any one of claims 1 to 5,
wherein the first binding site competes, for binding to LAG3, with an anti-LAG3 antibody that includes:
(i) a heavy chain variable region including a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
a light chain variable region including a light chain CDR1 including an amino acid sequence of SEQ ID NO: 12, a light chain CDR2 including an amino acid sequence of SEQ ID NO: 13 and a light chain CDR3 including an amino acid sequence of SEQ ID NO: 14, or
(ii) a heavy chain variable region including an amino acid sequence of SEQ ID NO: 7, and a light chain variable region including an amino acid sequence of SEQ ID NO: 8.

9. The immunosuppressant according to any one of claims 1 to 8, wherein the second binding site specifically binds to CD3.

10. The immunosuppressant according to claim 9, wherein the second binding site includes a heavy chain variable region and a light chain variable region of an anti-CD3 antibody.

11. The immunosuppressant according to claim 10,
wherein the second binding site includes:
a heavy chain variable region that includes a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
a light chain variable region that includes a light chain CDR1 including an amino acid sequence of SEQ ID NO: 20, a light chain CDR2 including an amino acid sequence of SEQ ID NO: 21, and a light chain CDR3 including an amino acid sequence of SEQ ID NO: 22.

12. The immunosuppressant according to claim 10 or 11,
wherein the second binding site includes:
a heavy chain variable region that includes an amino acid sequence having an identity of 90% or more with an amino acid sequence of SEQ ID NO: 15; and
a light chain variable region that includes an amino acid sequence having an identity of 90% or more with an amino acid sequence of SEQ ID NO: 16.

13. The immunosuppressant according to any one of claims 1 to 8, wherein the second binding site specifically binds to CD8.

14. The immunosuppressant according to claim 13, wherein the second binding site includes a heavy chain variable region and a light chain variable region of an anti-CD8 antibody.

15. The immunosuppressant according to claim 14,
wherein the second binding site includes:
a heavy chain variable region that includes a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
a light chain variable region that includes a light chain CDR1 including an amino acid sequence of SEQ ID NO: 28, a light chain CDR2 including an amino acid sequence of SEQ ID NO: 29, and a light chain CDR3 including an amino acid sequence of SEQ ID NO: 30.

16. The immunosuppressant according to claim 14 or 15,
wherein the second binding site includes:
a heavy chain variable region that includes an amino acid sequence having an identity of 90% or more with an amino acid sequence of SEQ ID NO: 23; and
a light chain variable region that includes an amino acid sequence having an identity of 90% or more with an amino acid sequence of SEQ ID NO: 24.

17. The immunosuppressant according to any one of claims 1 to 16, for prevention and/or treatment of an autoimmune disease, an allergic disease, or a graft-versus-host disease.

18. A method for preventing and/or treating an autoimmune disease, an allergic disease, or a graft-versus-host disease, the method including:
administering an effective amount of the immunosuppressant according to any one of claims 1 to 16, to a subject in need thereof.

19. The immunosuppressant according to any one of claims 1 to 16, for use in prevention and/or treatment of an autoimmune disease, an allergic disease, or a graft-versus-host disease.

20. A use of the immunosuppressant according to any one of claims 1 to 16 for production of a prophylactic and/or therapeutic agent for an autoimmune disease, an allergic disease, or a graft-versus-host disease.

21. A bispecific molecule including a first binding site that specifically binds to LAG3 and a second binding site that specifically binds to CD3 or CD8.

22. The bispecific molecule of claim 21, allowing binding of LAG3 to an MHC class II molecule.

23. The bispecific molecule according to claim 21 or 22, wherein the first binding site binds to a portion that is included in a D1 region of LAG3 and is not included in an extra loop of LAG3.

24. The bispecific molecule according to any one of claims 21 to 23, wherein the first binding site binds to a region including an amino acid/amino acids corresponding to asparagine at position 54, phenylalanine at position 55, valine at position 61, and/or isoleucine at position 62 of mouse LAG3 having an amino acid sequence of SEQ ID NO: 2.

25. The bispecific molecule according to any one of claims 21 to 24, wherein the first binding site includes a heavy chain variable region and a light chain variable region of an anti-LAG3 antibody.

26. The bispecific molecule according to claim 25,
wherein the first binding site includes:
a heavy chain variable region that includes a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
a light chain variable region that includes a light chain CDR1 including an amino acid sequence of SEQ ID NO: 12, a light chain CDR2 including an amino acid sequence of SEQ ID NO: 13, and a light chain CDR3 including an amino acid sequence of SEQ ID NO: 14.

27. The bispecific molecule according to claim 25 or 26,
wherein the first binding site includes:
a heavy chain variable region that includes an amino acid sequence having an identity of 90% or more with an amino acid sequence of SEQ ID NO: 7; and
a light chain variable region that includes an amino acid sequence having an identity of 90% or more with an amino acid sequence of SEQ ID NO: 8.

28. The bispecific molecule according to any one of claims 21 to 25,
wherein the first binding site competes, for binding to LAG3, with a first binding site of a bispecific molecule that includes:
(i) a heavy chain variable region including a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
a light chain variable region including a light chain CDR1 including an amino acid sequence of SEQ ID NO: 12, a light chain CDR2 including an amino acid sequence of SEQ ID NO: 13 and a light chain CDR3 including an amino acid sequence of SEQ ID NO: 14, or
(ii) a heavy chain variable region including an amino acid sequence of SEQ ID NO: 7, and a light chain variable region including an amino acid sequence of SEQ ID NO: 8.

29. The bispecific molecule according to any one of claims 21 to 28, wherein the second binding site specifically binds to CD3.

30. The bispecific molecule according to claim 29, wherein the second binding site includes a heavy chain variable region and a light chain variable region of an anti-CD3 antibody.

31. The bispecific molecule according to claim 30,
wherein the second binding site includes:
a heavy chain variable region that includes a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
a light chain variable region that includes a light chain CDR1 including an amino acid sequence of SEQ ID NO: 20, a light chain CDR2 including an amino acid sequence of SEQ ID NO: 21, and a light chain CDR3 including an amino acid sequence of SEQ ID NO: 22.

32. The bispecific molecule according to claim 30 or 31,
wherein the second binding site includes:
a heavy chain variable region that includes an amino acid sequence having an identity of 90% or more with an amino acid sequence of SEQ ID NO: 15; and
a light chain variable region that includes an amino acid sequence having an identity of 90% or more with an amino acid sequence of SEQ ID NO: 16.

33. The bispecific molecule according to any one of claims 21 to 28, wherein the second binding site specifically binds to CD8.

34. The bispecific molecule according to claim 33, wherein the second binding site includes a heavy chain variable region and a light chain variable region of an anti-CD8 antibody.

35. The bispecific molecule according to claim 34,
wherein the second binding site includes:
a heavy chain variable region that includes a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
a light chain variable region that includes a light chain CDR1 including an amino acid sequence of SEQ ID NO: 28, a light chain CDR2 including an amino acid sequence of SEQ ID NO: 29, and a light chain CDR3 including an amino acid sequence of SEQ ID NO: 30.

36. The bispecific molecule according to claim 34 or 35,
wherein the second binding site includes:
a heavy chain variable region that includes an amino acid sequence having an identity of 90% or more with an amino acid sequence of SEQ ID NO: 23; and
a light chain variable region that includes an amino acid sequence having an identity of 90% or more with an amino acid sequence of SEQ ID NO: 24.
